# EUROPEAN PATENT APPLICATION

(11) **EP 2 792 329 A2**
(43) Date of publication of application: **22.10.2014**
(21) Application number: 14165137.2
(22) Date of filing: 17.04.2014
(51) Int. Cl.: A61C 1/08

(54) **Surgical guide**

(30) Priority: 17.04.2013 GB 201306954
(71) Applicant: Digital Srl, 41042 Fiorano (Modena) (IT)
(72) Inventor: Albi, Claudio, 37131 Verona (IT); Giberti, Lorenzo, 40129 Bologna (IT)
(74) Representative: Openshaw, Paul Malcolm

(57) **Abstract**

A surgical guide comprising: a guide body configured to fit to a surface of a part of a human or animal body; and a cutting head guide that, when the surgical guide is fitted to the surface of the part of a human or animal body, defines a predetermined path across the surface from one point on the surface to a different point on the surface, the cutting head guide being for guiding a cutting head of a surgical device along the defined path.

## Description

### FIELD OF THE INVENTION

The present invention relates to surgical guides and the production thereof.

### BACKGROUND

When replacing a person's tooth with a prosthetic tooth, a hole is drilled in the person's jawbone, a dental implant is then inserted into the drilled hole, and a replacement tooth is then fixed to this dental implant.

The drilling of the hole in the patient's jawbone into which the dental implant is inserted is typically guided by a tube within a dental guide.

There are many known processes for producing dental guides, for example, a method used to produce the Materialise 'SurgyGuide'.

Such methods may, for example, comprise applying an X-ray opaque layer to a radiological guide, which is then inserted into the patient mouth before X-ray images of the patient's teeth are taken.

Also, such methods may use stereo lithography, otherwise known as rapid prototyping, to manufacture the guide from a digital computer model of the patient's jaw with the intended implants.

Other methods for producing dental guide include those described in UK Patent Application GB1117955.3.

In a separate field, piezoelectric ultrasound sources are used to perform piezoelectric surgery.

For example, US 6,695,847 discloses a piezoelectric surgical device and method for bone surgery. The disclosed device comprises a tip (or cutter) which is set in vibration at a modulated ultrasonic frequency. The device is particularly suitable for oral surgical procedures such as bone sampling, excision of cysts, third molar extraction, preparation of alveolar sites, creation of an opening into the maxillary sinus (Caldwell Luc), elevation of the maxillary sinus by the crestal route and orthopedic and neurosurgical procedures such as osteoplasty, ostectomy and osteotomy.

### SUMMARY OF THE INVENTION

In a first aspect, the present invention provides a surgical guide comprising: a guide body configured to fit to a surface of a part of a human or animal body; and a cutting head guide that, when the surgical guide is fitted to the surface of the part of a human or animal body, defines a predetermined path across the surface from one point on the surface to a different point on the surface, the cutting head guide being for guiding a cutting head of a surgical device along the defined path.

The surgical guide may be provided with a surgical device, e.g. a piezoelectric surgical device, adapted to be received and guided by the surgical guide. For example, the cutting head of a piezoelectric surgical device may be adapted to be received and guided by the surgical guide or vice versa.

The part of a human or animal body may be at least part of a dental arch of the human or animal. Alternatively, the part of a human or animal body may be a part of a human or animal body other than a dental arch of the human or animal.

The surgical device may be a piezoelectric surgical device. The surgical device may be a surgical device other than a drill.

The cutting head guide may comprise a channel through the guide body, the channel being such that, when the surgical guide is fitted to the surface of the part of a human or animal body, a cutting head of a surgical device may be moved along the channel from a first position to a second position. The first position may be a position in which the cutting head of a surgical device is not in contact with the surface of the part of a human or animal body. The second position may be a position in which the cutting head of a surgical device is in contact with the surface of the part of a human or animal body.

The channel may be such that, when the surgical guide is fitted to the surface of the part of a human or animal body and when the cutting head of a surgical device is in the second position, the cutting head of a surgical device may be moved along the path guided by the channel.

The channel may have a non-round cross-section in a plane through which the cutting head is moved when the cutting head is moved along the path.

The channel may be a slit in the surgical guide. Each slit may be a substantially straight slit.

The guide body may comprise a structure that is complementary to the surface of a part of a human or animal body.

In a further aspect, the present invention provides a surgical guide comprising: a guide body configured to fit to a part of a human or animal body, and a channel through the guide body; wherein the channel is such that, when the surgical guide is fitted to the surface of the part of a human or animal body, a cutting head of a surgical device may be moved along the channel from a first position to a second position; the first position is a position in which the cutting head of a surgical device is not in contact with the part of a human or animal body; the second position is a position in which the cutting head of a surgical device is in contact with the part of a human or animal body; and the channel has a non-round cross section (e.g. in the plane that is perpendicular to the direction in which the cutting head of a surgical device moves when the cutting head of a surgical device is moved along the channel from a first position to a second position).

The surgical guide may be provided with a surgical device, e.g. a piezoelectric surgical device, adapted to be received and guided by the channel of the surgical guide. For example, the cutting head of a piezoelectric surgical device may be adapted to be received and guided by the channel or vice versa.

The part of a human or animal body may be at least part of a dental arch of the human or animal. Alternatively, the part of a human or animal body may be a part of a human or animal body other than a dental arch of the human or animal.

The surgical device may be a piezoelectric surgical device. The surgical device may be a surgical device other than a drill.

When the surgical guide is fitted to the part of a human or animal body, the channel may define a predetermined path across the surface of the part of a human or animal body from one point on the surface to a different point on the surface. The channel may be for guiding, along the defined path, a cutting head of a surgical device that has been moved along the channel from the first position to the second position.

The channel may be a slit in the guide body. Each slit may be a substantially straight slit.

The guide body may comprise a structure that is complementary to the surface of a part of a human or animal body.

In a further aspect, the present invention provides a method of manufacturing a surgical guide. The surgical guide may be in accordance with any of the above aspects. The method comprises: providing an assembly, the assembly comprising: a first positive physical model of a part of a human or animal body, the first positive physical model comprising a location indicating structure corresponding to a predetermined location in the part of a human or animal body; and a location indicating member coupled to the location indicating structure; coating at least part of the assembly with a coating material; removing, from the coated assembly, the location indicating member; and removing, from the first positive physical model, the coating material, thereby producing the surgical guide.

The location indicating structure may correspond to a predetermined path across a surface of the part of a human or animal body.

The part of a human or animal body may be at least part of a dental arch of the human or animal. Alternatively, the part of a human or animal body may be part of the human or animal body other than a dental arch of the human or animal.

The location indicating structure may be any location indicating structure other than a bore.

The step of providing the assembly may comprise: providing the first positive physical model; and coupling, to the location indicating structure of the first positive physical model, a location indicating member.

The location indicating structure may be a cavity located in the first positive physical model at a location that corresponds to the predetermined location in the part of a human or animal body.

The step of coupling may comprise inserting the location indicating member into the cavity.

The cavity may be a slit in the first positive physical model.

The location indicating member may be a sheet of material.

The step of providing the first positive physical model may comprise: producing a first digital model, the first digital model being a digital model of the internal structure of the part of a human or animal body; specifying, in the digital model, the predetermined location in the part of a human or animal body, thereby producing a second digital model; producing a first negative physical model of the part of a human or animal body; using the first negative physical model, producing a second positive physical model of the part of a human or animal body; producing a third digital model, the third digital model being a digital model of the second positive physical model; registering the second digital model and the third digital model, thereby producing a fourth digital model; and, using the fourth digital model, producing the first positive physical model.

The step of, using the fourth digital model, producing the first positive physical model may comprise: using the fourth digital model, controlling a cutting machine to cut the second positive physical model so as to form the one or more location indicating structures in the second positive physical model, thereby producing the first positive physical model.

The step of, using the fourth digital model, producing the first positive physical model may comprise: using the fourth digital model, producing a mould for the first positive physical model; and using the mould, producing the first positive physical model.

The mould may comprise a first mould part and a second mould part.

The step of producing the mould may comprise: producing a second negative physical model of the part of a human or animal body, thereby providing the first mould part; and, using the fourth digital model, producing the second mould part.

The second mould part may comprise: a base portion; and one or more projections extending from the base portion, each projection defining a respective location indicating structure in the first positive physical model.

Each projection may be any projection other than a hollow tube.

The step of producing a first digital model may comprise: capturing one or more X-ray images of the part of a human or animal body; and producing the digital model using said one or more X-ray images.

The step of producing the first negative physical model may comprise direct impression modelling of the part of a human or animal body.

In a further aspect, the present invention provides a mould part for a mould, the mould being for producing a positive physical model of part of a human or animal body, the positive physical model comprising one or more location indicating structures, the location of each of the location indicating structure in the positive physical model being substantially the same as a respective predetermined location in the part of the human or animal body, the mould part comprising: a base portion; and one or more projections extending from the base portion; wherein each projection defines a respective location indicating structure in positive physical model.

Each location indicating structure may correspond to a predetermined path across a surface of the part of a human or animal body.

Each predetermined location in the part of the human or animal body may be a predetermined path across a surface of the part of a human or animal body.

The part of the human or animal body may be at least part of a dental arch of the patient. Alternatively, the part of a human or animal body may be part of the human or animal body other than a dental arch of the patient.

Each predetermined location in the part of the human or animal body may be a location in the part of the human or animal body at which the part of the human or animal body is to be cut using a surgical device.

The surgical device may be any surgical device other than a dental drill.

The surgical device may be a piezoelectric surgical device.

Each location indicating structure may be any location indicating structure other than a bore.

Each projection may be any projection other than a hollow tube.

One or more of the projections may comprise respective cavities, each cavity being accessible via an opening, such that each projection is adapted to receive a location indicating member.

One or more of the openings may be a slit. Each slit may be a substantially straight slit.

In a further aspect, the present invention provides a method of manufacturing a mould part. The mould part may be in accordance with the preceding aspect. The method comprises: producing a first digital model, the first digital model being a digital model of the internal structure of the part of the human or animal body; specifying, in the digital model, each of the predetermined locations in the part of the human or animal body, thereby producing a second digital model; producing a negative physical model of the part of the human or animal body; using the negative physical model, producing a positive physical model of the at part of the human or animal body; producing a third digital model, the third digital model being a digital model of the positive physical model; registering the second digital model and the third digital model, thereby producing a fourth digital model; and using the fourth digital model, controlling a cutting machine to cut a work piece into the shape of the mould part.

The part of the human or animal body may be at least part of a dental arch of the patient. Alternatively, the part of a human or animal body may be part of the human or animal body other than a dental arch of the patient.

The step of producing a first digital model may comprise: capturing one or more X-ray images of the part of the human or animal body, and producing the digital model using said one or more X-ray images.

The step of producing the negative physical model may comprise direct impression modelling of the part of the human or animal body.

In a further aspect, the present invention provides a method of manufacturing a surgical guide, the method comprising: providing a mould for producing a positive physical model of part of a human or animal body, the mould comprising a mould part, the mould part being in accordance with any of the above aspects, and a further mould part, the further mould part being a negative physical model of the part of the human or animal body; using the mould, producing a positive physical model of the part of the human or animal body, the positive physical model comprising one or more location indicating structures, the location of each of the location indicating structure in the positive physical model being substantially the same as a respective predetermined location in the part of the human or animal body; coupling, to each of the location indicating structures of the first positive physical model, a respective location indicating member, thereby providing an assembly; coating at least part of the assembly with a coating material; removing, from the coated assembly, the one or more indicating members; and removing, from the positive physical model, the coating material, thereby producing the surgical guide.

The part of the human or animal body may be at least part of a dental arch of the patient. Alternatively, the part of the human or animal body is part of the human or animal body other than a dental arch of the patient.

The step of providing a mould part may comprise manufacturing the mould part using a method in accordance with any of the above aspects.

In a further aspect, the present invention provides a method of manufacturing a surgical guide, the method comprising: producing a first digital model, the first digital model being a digital model of the internal structure of part of the human or animal body; specifying, in the digital model, one or more predetermined locations in the part of the human or animal body, thereby producing a second digital model; producing a positive physical model of the part of the human or animal body; producing a third digital model, the third digital model being a digital model of the positive physical model; registering the second digital model and the third digital model, thereby producing a fourth digital model; using the fourth digital model, controlling a cutting machine to cut the positive physical model so as to form one or more location indicating structures in the positive physical model, each location indicating structure in the positive physical model corresponding to a respective one of the predetermined locations in the part of the human or animal body; coupling, to each of the location indicating structures formed in the first positive physical model, a respective location indicating member, thereby providing an assembly; coating at least part of the assembly with a coating material; removing, from the coated assembly, the one or more indicating members; and removing, from the positive physical model, the coating material, thereby producing the surgical guide.

Specifying, in the digital model, one or more predetermined locations in the part of the human or animal body may comprise specifying, in the digital model, one or more paths across a surface of the part of a human or animal body.

The part of the human or animal body may be at least part of a dental arch of the patient. Alternatively, the part of the human or animal body may be part of the human or animal body other than a dental arch of the patient.

The step of producing a positive physical model of the part of the human or animal body may comprises producing a negative physical model of the at least part of the part of the human or animal body, and using the negative physical model, producing the positive physical model.

Each predetermined location in the part of the human or animal body may be a location in the part of the human or animal body at which the part of the human or animal body is to be cut using a surgical device.

The surgical device may be any surgical device other than a dental drill. The surgical device may be a piezoelectric surgical device.

Each location indicating structure may be any location indicating structure other than a bore.

One or more of the location indicating structures may comprise respective cavities, each cavity being accessible via an opening, such that each cavity is adapted to receive a location indicating member.

The step of coupling, to each of the location indicating structures formed in the first positive physical model, a respective location indicating member may comprise inserting a location indicating member into each of the one or more cavities.

One or more of the openings may be a slit. Each slit may be a substantially straight slit.

For each slit, the location indicating member that is inserted into that slit may be a sheet of material.

### BRIEF DESCRIPTION OF THE DRAWINGS

Figure 1 is a schematic illustration (not to scale) of a guide for a piezoelectric surgical device;
Figure 2 is a schematic illustration (not to scale) showing certain pieces of apparatus used in a process of manufacturing the guide;
Figure 3 is a process flow chart showing certain steps of an embodiment of a process of manufacturing the guide;
Figure 4 is a schematic illustration (not to scale) of a perspective view of a dental fork;
Figure 5 is a schematic illustration (not to scale) of an exploded view of an assembly formed during the process of Figure 3;
Figure 6 is a schematic illustration (not to scale) of a side view of certain elements of a surgery planning model formed during the process of Figure 3;
Figure 7 is a schematic illustration (not to scale) of a digital model of a base plate formed during the process of Figure 3;
Figure 8 is a schematic illustration (not to scale) of an exploded view of an assembly formed during the process of Figure 3;
Figure 9 is a schematic illustration (not to scale) of an exploded view of an assembly formed during the process of Figure 3;
Figure 10 is a schematic illustration (not to scale) showing a plaster cast coupled to a base plate;
Figure 11 is a schematic illustration (not to scale) showing a plaster cast coupled to a base plate, and a plurality of metal sheets;
Figure 12 is a schematic illustration (not to scale) of the structure shown in Figure 11 with resin applied to it; and
Figure 13 is a process flow chart showing certain steps of a further embodiment of a process of producing a guide.

### DETAILED DESCRIPTION

It will be appreciated that relative terms such as front and back, upper and lower and so on, are used below merely for ease of reference to the Figures, and these terms are not limiting as such, and any differing directions or positions and so on may be implemented. In particular, for convenience, in the Figures, surfaces of objects that are towards the top of a page are typically referred to as the "top" or "upper surface" of that object. Similarly. surfaces of objects that are towards the bottom of a page are typically referred to as the "bottom" or "lower surface" of that object, and so on.

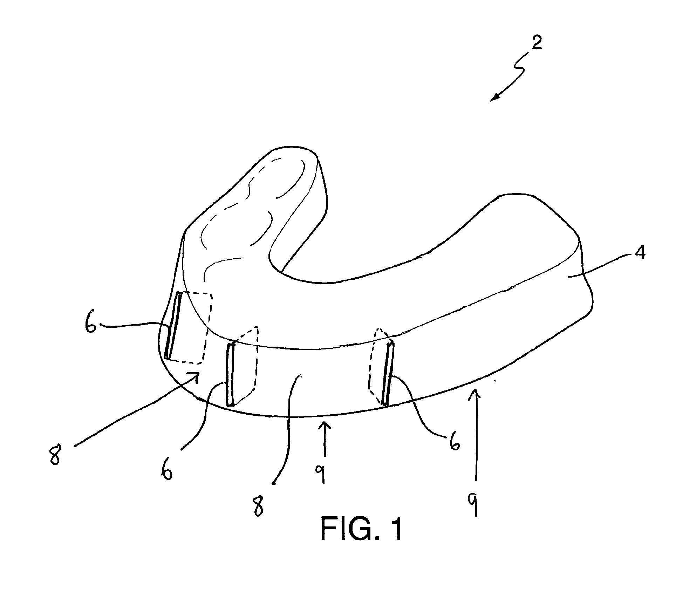

Figure 1 is a schematic illustration (not to scale) of a guide for a piezoelectric surgical device, hereinafter referred to as the "guide" and indicated in Figure 1 by the reference numeral 2.

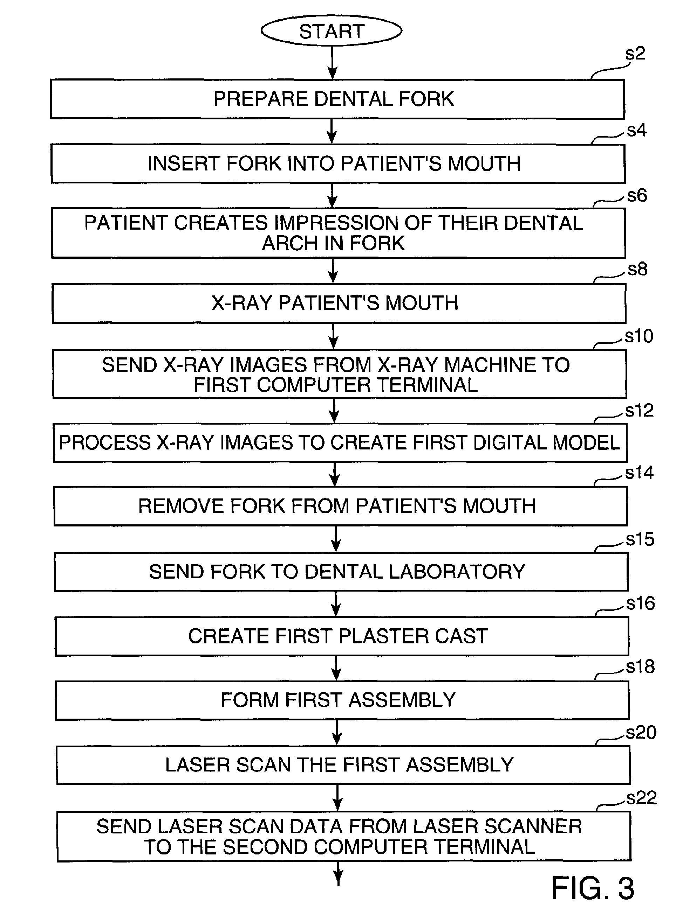

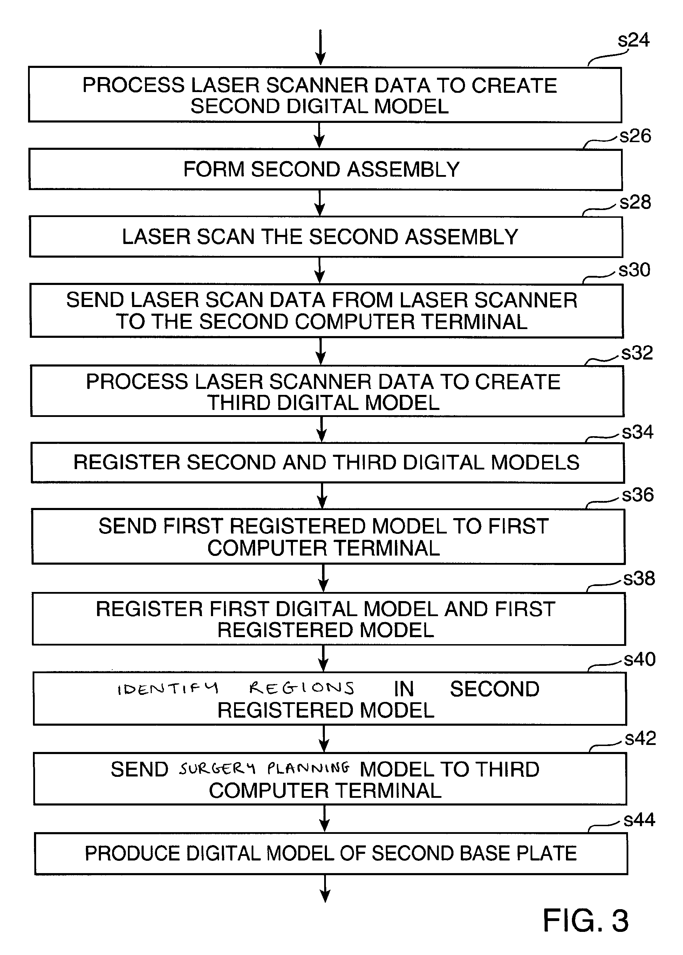

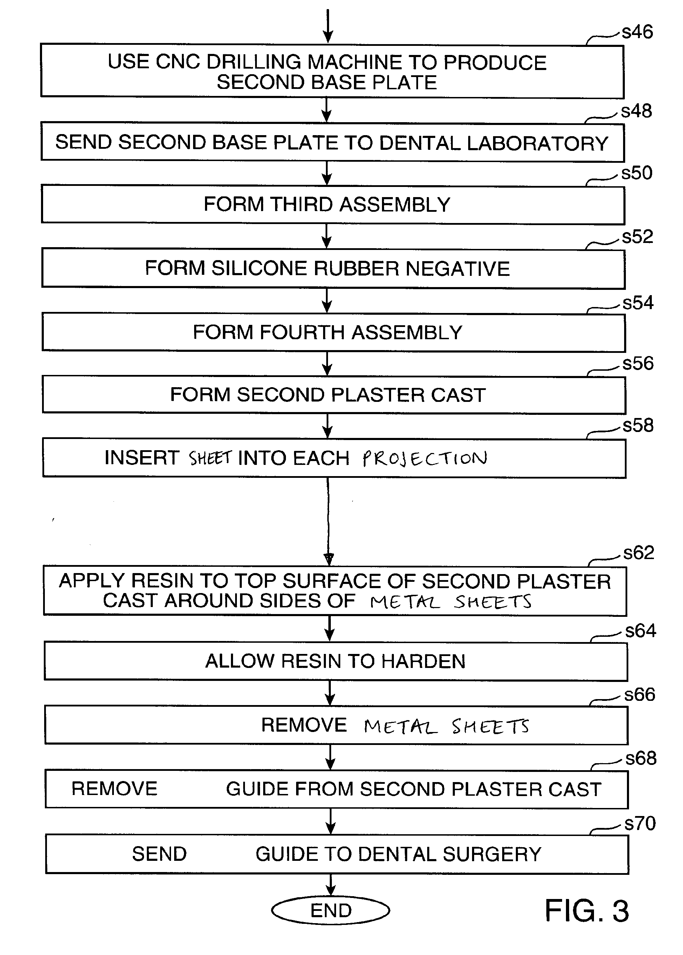

An embodiment of a process of manufacturing the guide 2 is described in more detail later below with reference to Figure 3.

In this embodiment, the guide 2 comprises a guide body 4.

In this embodiment, the guide 2 further comprises three guide slits 6. Each of the guide slits 6 is a relatively long, thin slit through the entire thickness of a front surface 8 of the guide 2. Thus, the slits 6 are relative thin channels or passages through the entire thickness of a front surface 8 of the guide 2.

The production of the guide 2 is described in more detail later below with reference to Figure 3.

In this embodiment, a lower surface 9 of the guide body 4 is complementary to at least part of a dental arch of a patient. The slits 6 pass through the guide body 4, from the front surface 8 of the guide body 4 (which is opposite to the lower surface 9), to the lower surface 9.

As described in more detail later below, in use, the guide 2 is inserted into the mouth of a patient for which the guide 2 has been produced such that the lower surface 9 of the guide body 4 fits against the lower dental arch of the patient. A dental surgeon then inserts at least the cutting head of a piezoelectric surgical device through each of the guide slits 6 in turn (from the front surface 8 to the lower surface 9, and into the patient's jawbone), so as to cut into the patient's jawbone at a predetermined position.

In this embodiment, the slits 6 are for guiding the movement of the cutting head of the piezoelectric surgical device). Each of the slits 6 defines, on the dental arch of the patient, a path across the surface of the dental arch along which the cutting head of the piezoelectric surgical device is guided. In this embodiment, as the slits 6 are straight slits, the paths on the dental arch of the patient are straight paths. However, in other embodiments, the paths defined by the slits may be non-straight e.g. curved. In this embodiment, a path on the dental arch defined by a slit 6 is from one point on the dental arch to a different point on the dental arch.

In some embodiments, the slits 6 do not define a path across the surface of the dental arch. For example, the cross section of a slit may be the same size and shape as cross section of the cutting head of the piezoelectric surgical device (which may, for example, be rectangular).

In this embodiment, in use, the slits 6 guide the cutting head of a piezoelectric surgical device in a first direction. The first direction is a direction that points from the front surface 8 to the lower surface 9 and into the jawbone of the patient.

Also, in this embodiment, the slits 6 guide the cutting head of a piezoelectric surgical device in at least one further direction, e.g. a direction that is perpendicular to the first direction. In particular, in this embodiment, each of the slits 6 guide the cutting head of a piezoelectric surgical device such that the cutting head moves along a line which is perpendicular to the first direction. This line along which the cutting head of a piezoelectric surgical device is guided may be a straight line, e.g. if the guiding slit 6 is a straight slit.

One example of a piezoelectric surgical device is that disclosed in US 6,695,847, which is incorporated herein by reference.

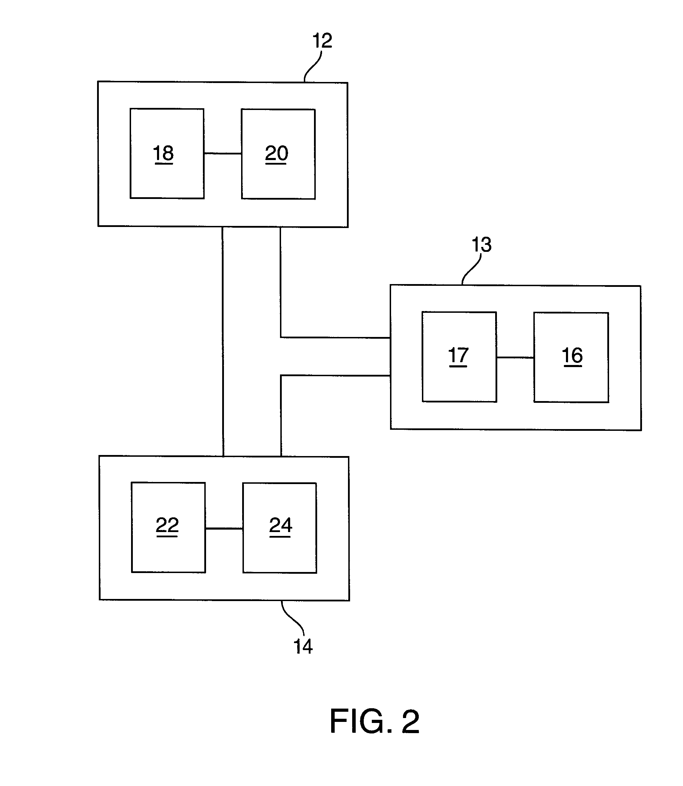

Figure 2 is a schematic illustration (not to scale) showing certain pieces of apparatus used in the below described process of manufacturing the guide 2.

In this embodiment, the pieces of apparatus used in the below described process of manufacturing the guide 2 are located at three separate (i.e. remote from one another) locations. These locations are the surgery of the dental surgeon (hereinafter referred to as the "dental surgery" and indicated in figure 2 by the reference numeral 12), a dental laboratory 13, and a computer numerical control (CNC) drilling facility 14.

In this embodiment, the dental surgery 12 comprises an X-ray machine 18, and a first computer terminal 20.

In this embodiment, the X-ray machine 18 is a conventional dental X-ray device capable of capturing X-ray images of a patient's jawbones and teeth. The X-ray machine 18 is coupled to the first computer terminal 20 such that, in operation, image data measured by the X-ray machine 18 are sent from the X-ray machine 18 to the first computer terminal 20.

In this embodiment, the first computer terminal 20 is arranged to process image data received from the X-ray machine 18. The processing of the received image data performed by the first computer terminal 20 is described in more detail later below with reference to Figure 3.

In this embodiment, the dental laboratory 13 comprises a laser scanner 16 and a second computer terminal 17.

In this embodiment, the laser scanner 16 is a conventional laser scanner capable of scanning three-dimensional objects. The laser scanner 16 is coupled to the second computer terminal 17 such that, in operation, image data measured by the laser scanner 16 are sent from the laser scanner 16 to the second computer terminal 17.

In this embodiment, the second computer terminal 17 is arranged to process image data received from the laser scanner 16. The processing of the received image data performed by the second computer terminal 17 is described in more detail later below with reference to Figure 3.

In this embodiment, CNC drilling facility 14 comprises a third computer terminal 22 and a CNC drilling machine 24.

In this embodiment, the CNC drilling machine 24 is a conventional CNC drilling machine 24. The CNC drilling machine 24 is coupled to the third computer terminal 22 such that, in operation, the CNC drilling machine 24 may be controlled using the third computer terminal 22.

In this embodiment, the dental surgery 12, the dental laboratory 13, and the CNC drilling facility 14 are coupled together such that, objects may be transported (e.g. by post or courier) between those entities, and such that data (e.g. digital files) may be sent (e.g. via email or the Internet) between those entities.

Figure 3 is a process flow chart showing certain steps of an embodiment of a process of manufacturing the guide 2.

In this embodiment, the guide 2 is for facilitating dental surgery including the cutting of a lower jawbone of a patient. In this embodiment, the patient is an adult male human.

In this embodiment, steps s2 to s14 are performed at the dental surgery 12, step s15 is performed between the dental surgery 12 and the dental laboratory 13, steps s16 to s34 are performed at the dental laboratory 13, step s36 is performed between the dental laboratory 13 and the dental surgery 12, steps s38 and s40 are performed at the dental surgery 12, step s42 is performed between the dental surgery 12 and the CNC drilling facility 14, steps s44 and s46 are performed at the CNC drilling facility 14, step s48 is performed between the CNC drilling facility 14 and the dental laboratory 13, steps s50 to s68 are performed at the dental laboratory 13, and step s70 is performed between the dental laboratory 13 and the dental surgery 12.

At step s2, a "dental fork", hereinafter referred to as "the fork", is prepared.

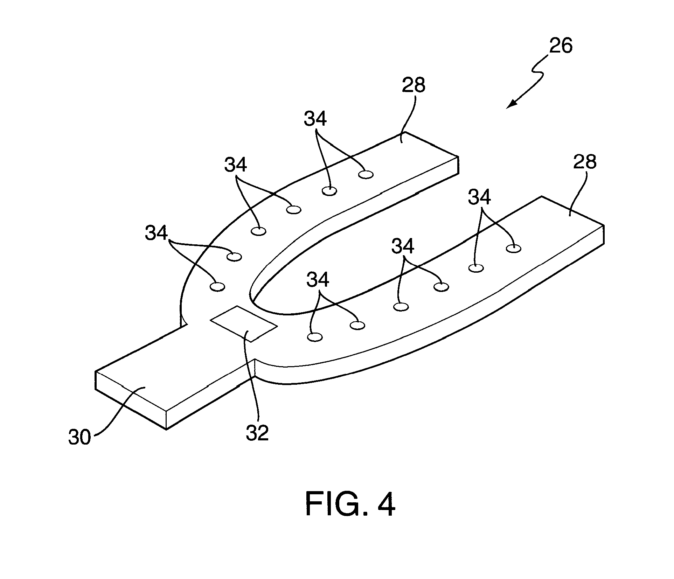

Figure 4 is a schematic illustration (not to scale) of a perspective view of the fork 26 used in this embodiment.

In this embodiment, the fork 26 is made of a biocompatible material.

In this embodiment, the fork 26 comprises a U-shaped section. The arms of the U-shaped section of the fork 26 are indicated in figure 4 by the reference numerals 28.

The fork 26 further comprises a handle 30 which protrudes from the bottom of the U-shaped section away from the arms 28.

In this embodiment, the fork 26 is of a size such that the U-shaped portion of the fork 26 may be inserted into the patient's mouth. Whilst the U-shaped portion is in the patient's mouth, the handle 30 protrudes from the patient's mouth.

In this embodiment, the arc of the U-shaped portion is substantially the same shape as a dental arch (i.e. the arc formed by a row of teeth) of an average male human (i.e. a generic patient).

In this embodiment, the fork 26 comprises a fiduciary marker 32. In this embodiment, the fiduciary marker 32 is a single component or feature that can be used as a reference in an image, e.g. a single 3D bar code.

In this embodiment, the fiduciary marker 32 is positioned relatively centrally on the fork 26, e.g. the fiduciary marker is positioned at or proximate to the centre of mass of the body of the fork 26.

In this embodiment, the fiduciary marker 32 is detectable by both the laser scanner 16 and the X-ray machine 18. The fiduciary marker 32 is made of a biocompatible and substantially radio-opaque material.

In this embodiment, the fork 26 further comprises a plurality of holes 34. The holes 34 are positioned along the length of the arms 28 of the U-shaped portion of the fork 26. Furthermore, each of the holes 34 is through the entire thickness of an arm 28, i.e. from a top surface of the fork 26 to a bottom surface 26 of the fork 26.

In this embodiment, the length of each of the arms 28 of the fork 26 is 60mm. Also, the length of the handle 30 of the fork 26 is 30mm. Also, the thickness of the fork 26 is 6mm. In other embodiments, the fork 26 may have different dimensions.

In this embodiment, at step s2 the fork 26 is prepared (e.g. by a dental technician or assistant to the dental surgeon or dentist) by applying silicone rubber to the top and bottom surface of the U-shaped portion of the fork 26. The silicone rubber is applied such that silicone rubber is forced through the entire length of each of the holes 34. Thus, the silicone rubber applied to the top surface of the U-shaped portion is in contact (via the holes 34) with the silicone rubber applied to the bottom of the U-shaped portion. This advantageously tends to provide that the coupling of the silicone rubber to the top and bottom surfaces of the U-shaped portion of the fork 26 is relatively secure.

Furthermore, the holes 34 tend to increase the surface area of the top and bottom surfaces of the U-shaped portion to which the silicone rubber is applied. This also advantageously tends to provide that the coupling of the silicone rubber to the top and bottom surfaces of the U-shaped portion of the fork 26 is relatively secure.

At step s4, the prepared fork 26 (i.e. the fork 26 and the silicone rubber applied to the fork 26) is inserted into the patient's mouth.

In this embodiment, the prepared fork 26 is positioned in the patient's mouth such that the silicone rubber applied to the top surface of the fork 26 is in contact with the upper dental arch of the patient (i.e. the patient's teeth, or gum where teeth should be located, on the patient's upper jaw).

Also, in this embodiment, the prepared fork 26 is positioned in the patient's mouth such that the silicone rubber applied to the bottom surface of the fork 26 is in contact with the lower dental arch of the patient (i.e. the patient's teeth, or gum where teeth should be located, on the patient's lower jaw).

At step s6, the patient bites down on the prepared fork 26, thereby creating an impression of the dental arch of the patient in the silicone rubber.

In particular, an impression of the upper dental arch of the patient is created in the silicone rubber that has been applied to the top surface of the fork 26.

Also, an impression of the lower dental arch of the patient is created in the silicone rubber that has been applied to the bottom surface of the fork 26.

At step s8, whilst the fork 26 is still in the patient's mouth and the patient is still biting down on the fork 26, one or more X-ray images of the patient's mouth are taken using the X-ray machine 18.

In this embodiment, a conventional cone-beam reconstruction process is used to capture the images of the patient's mouth.

In this embodiment, the X-ray images taken at step s8 capture the patient's teeth, the patient's jawbones, and the fork 26 (including the fiduciary marker 32 of the fork 26). The relative positions of these entities are also captured in the X-ray images.

In this embodiment, the one or more X-ray images of the patient's mouth are sufficient to allow for a 3-dimensional digital model of the patient's teeth, the patient's jawbones, and the fork 26 to be created.

In this embodiment, the resolution of each X-ray image is 512 x 512 pixels for each image.

At step s10, the captured X-ray images (or data corresponding to the X-ray images) are sent from the X-ray machine 18 to the first computer terminal 20.

In this embodiment, the Digital Imaging and Communications in Medicine (DICOM) format is adopted for the handling, storing, printing, and transmitting of the X-ray images.

At step s12, the first computer terminal 20 processes the received X-ray images to create a first 3-dimensional digital model. The first digital model comprises digital representations of the patient's teeth, the patient's jawbones, and the fork 26.

At step s14, the fork 26 with attached the silicone rubber is removed from the patient's mouth.

At step s15, the fork 26 is sent from the dental surgery 12 to the dental laboratory 13.

At step s16, from the impression of the patient's lower dental arch in the silicone rubber attached to the bottom surface of the U-shaped portion of the fork 26, a plaster cast of the patient's lower dental arch, hereinafter referred to as the "first plaster cast", is created at the dental laboratory 13.

The first plaster cast is a 3-dimensional plaster model of the patient's lower dental arch. In other embodiment, this model of the patient's lower dental arch is produced in a different appropriate way, for example as described in the further embodiment later below.

At step s18, a base plate (hereinafter referred to as the "first base plate"), the first plaster cast, and the fork 26 (with the indented silicone rubber) are coupled together to form a first assembly.

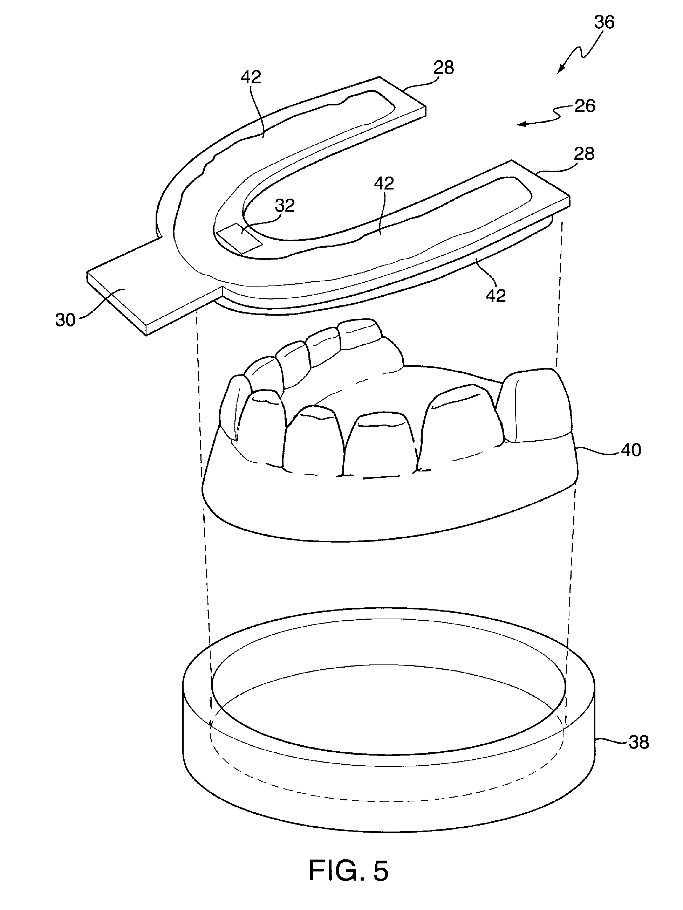

Figure 5 is a schematic illustration (not to scale) of an exploded view of the first assembly 36 formed at step s18 of the process of Figure 3.

In this embodiment, the first assembly 36 comprises the first base plate 38, the first plaster cast 40, and the fork 26. The indented silicone rubber 42 is attached to the fork 26.

In this embodiment, the first base plate 38 is a conventional plastic or resin base plate. In this embodiment, the shape of the first base plate 38 is that of a tube having one end of the tube sealed. In this embodiment, the internal diameter of the tube portion of the first base plate 38 is such that the first plaster cast 40 fits securely inside the tube portion of the first base plate 38.

In this embodiment, the first plaster cast 40 is positioned on a top surface of the first base plate 38, i.e. on the sealed end of the first base plate 38 such that the first plaster cast 40 is at least partly inside the tube portion of the first base plate 38. This is done such that a top surface of the first plaster cast 40 (i.e. the surface of the first plaster cast 40 that is the shape of the patient's lower dental arch) is not in contact with the first base plate 38. Also, this is done such that a bottom surface of the first plaster cast 40 (i.e. the surface of the first plaster cast 40 that is opposite to the top surface of the first plaster cast 40) is in contact with the first base plate 38.

The first plaster cast 40 is positioned such that the first plaster cast does not slide about on the top surface of the first base plate 38, i.e. the relative positions of the first plaster cast 40 and the first base plate 38 are substantially fixed.

In this embodiment, the fork 26 (and attached silicone rubber 42) is positioned on to the top of the first plaster cast 40. This is done such that the silicone rubber 42 attached to the bottom surface of the fork 26, into which an indentation of the patient's lower dental arch has been formed, is in contact with the top surface of the first plaster cast 40 (i.e. the surface of the first plaster cast 40 that is the shape of the patient's lower dental arch). Furthermore, this is done such that the negative indentations in the silicone rubber 42 (made by the patient's lower dental arch) mate with the complementary surface of the first plaster cast 40 (i.e. the surface of the first plaster cast 40 having positive protrusions corresponding to the negative indentations in the silicone rubber 42).

The above described relative placement of the first base plate 38, the first plaster cast 40, and the fork 26 is indicated in Figure 5 by dotted lines.

At step s20, the first assembly 36 is scanned using the laser scanner 16.

In this embodiment, data produced by the scanning of the first assembly 36 by the laser scanner 16 are sufficient to allow for a 3-dimensional digital model of the first assembly 36 to be created.

At step s22, data from the laser scanner 16 corresponding to the scanned first assembly 36 are transferred from the laser scanner 16 to the second computer terminal 17.

At step s24, the second computer terminal 17 processes the received laser scanner data to create a second 3-dimensional digital model. The second digital model comprises digital representations of the fork 26, the first plaster cast 40, and the first base plate 38.

At step s26, the first base plate 38 and the first plaster cast 40 are coupled together to form a second assembly.

In this embodiment, in the second assembly, the first plaster cast 40 is positioned on a top surface of the first base plate 38 in the same way as in the first assembly 36, i.e. the second assembly is the same as the first assembly with the fork 26 removed.

At step s28, the second assembly is scanned using the laser scanner 16.

At step s30, data from the laser scanner 16 corresponding to the scanned second assembly 36 are transferred from the laser scanner 16 to the second computer terminal 17.

At step s32, the second computer terminal 17 processes the received laser scanner data to create a third 3-dimensional digital model. The third digital model comprises digital representations of the first plaster cast 40, and the first base plate 38.

At step s34, the second digital model (created at step s24) and the third digital model (generated at step s32) are registered by the second computer terminal 17. In this embodiment, this registration process comprises aligning the digital models using surfaces common to both second and third digital models.

The registered second and third digital models are hereinafter referred to as the "first registered model". The first registered model comprises laser scanned images of the first base plate 38, first plaster cast 40, and the fork 26 (with the fiduciary marker 32).

At step s36, the first registered model is sent from the second computer terminal 17 (at the dental laboratory 13) to the first computer terminal 20 (at the dental surgery 12).

In this embodiment, the first registered model is sent between the computer terminals 17, 20 over the Internet.

At step s38, the first digital model (generated at step s12) and the first registered model (generated at step s34) are registered using the first computer terminal 20 (e.g. by the dental surgeon or an assistant to the dental surgeon).

In other words, at step s38 the first digital model and the first registered model are transformed into a common coordinate system.

In this embodiment, this image registration is performed by transforming one or more of the models such that the position and orientation of the fiduciary marker 32 in each of the digital models is substantially the same.

Thus, a 3-dimensional digital model comprising digital representations of the patient's teeth, the patient's jawbones, the fork 26, the first plaster cast 40 and the first base plate 38 is produced. This 3-dimensional digital model is hereinafter referred to as the "second registered model".

The image of the fork 26 within the second registered model may be removed from the second registered model.

At step s40, using the first computer terminal 20, the dental surgeon (or a different party) identifies regions in the second registered model that correspond to those regions in the patient's lower jawbone that are to be cut into using the piezoelectric surgical device. The identified regions are hereinafter referred to as "the regions".

In this embodiment, the identifying of the regions comprises the dental surgeon specifying, using the second registered model, data such as a depth, length, width, position, and orientation (e.g. with respect to the patient's lower jawbone) for each region.

Thus, at step s40 a 3-dimensional digital model comprising digital representations of the patient's teeth, the patient's jawbones, the fork 26 (optionally), the first plaster cast 40, the first base plate 38, and the regions is produced. This digital model is hereinafter referred to as the "surgery planning model".

Any appropriate software package may be used to manipulate digital models and e.g. perform step s40. For example, the "Geomagic", or "MIMICS" software packages may be used.

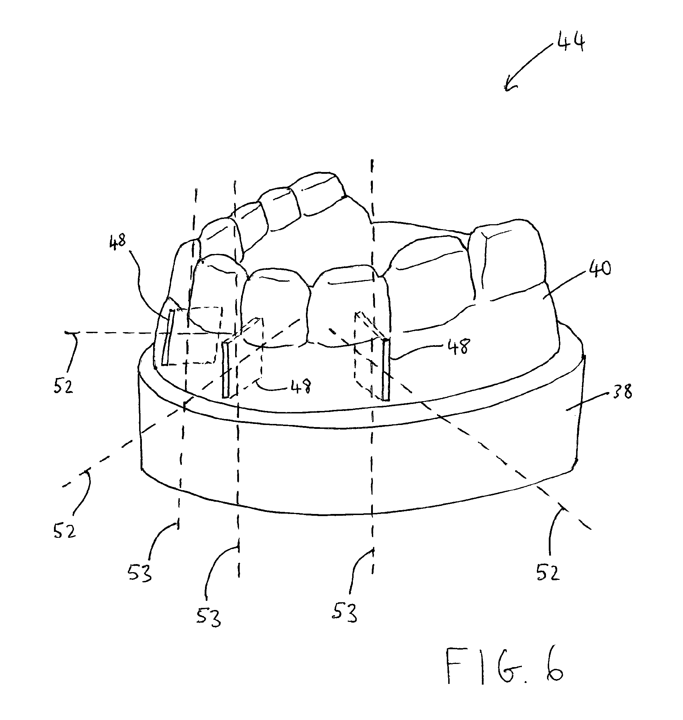

Figure 6 is a schematic illustration (not to scale) of a side view of certain elements of the surgery planning model 44. For purposes of clarity, digital representations of the fork 26 and the patient's jawbone is not shown in Figure 6. This side view of the surgery planning model 44 is a side view of the digital model as may be viewed e.g. on a computer display of the first computer terminal 20.

For convenience and ease of understanding, in Figure 6 the digital representation of the first base plate is indicated with the reference numeral corresponding to the first base plate (i.e. the reference numeral 38). Also, in Figure 6 the digital representation of the first plaster cast is indicated with the reference numeral corresponding to the first plaster cast (i.e. the reference numeral 40).

The regions identified by the dental surgeon (or other party) at step s40 are indicated in Figure 6 by the reference numeral 48.

In this embodiment, at step s40, the regions 48 are positioned and orientated in the surgery planning model 44 such that they correspond to the portions of the patient's jawbone that the dental surgeon wishes to cut using the piezoelectric surgical device. Furthermore, the lengths, positions, and orientations of the regions 48 may be altered so as to, for example, avoid nerves etc.

In this embodiment, each of the regions 48 is substantially cuboid in shape. In particular, each of the regions 48 is a slit-shaped, or slot-shaped regions. In other words, the regions 48 are relatively thin, relatively long regions having any appropriate depth. In other embodiments, one or more of the regions 48 may have a different appropriate shape.

A longitudinal axis for each of the regions 48 is shown in Figure 6 as a dotted line and indicated by the reference numeral 52.

A transverse axis for each of the regions 48 is shown in Figure 6 as a dotted line and indicated by the reference numeral 53.

At step s42, the surgery planning model 44 is sent from the first computer terminal 20 (at the dental surgery 12) to the third computer terminal 22 (at the CNC drilling facility 14).

In this embodiment, the surgery planning model 44 is sent between the computer terminals 20 and 22, over the Internet.

At step s44, the surgery planning model 44 is processed by the third computer terminal 22 to produce a digital model of a second base plate.

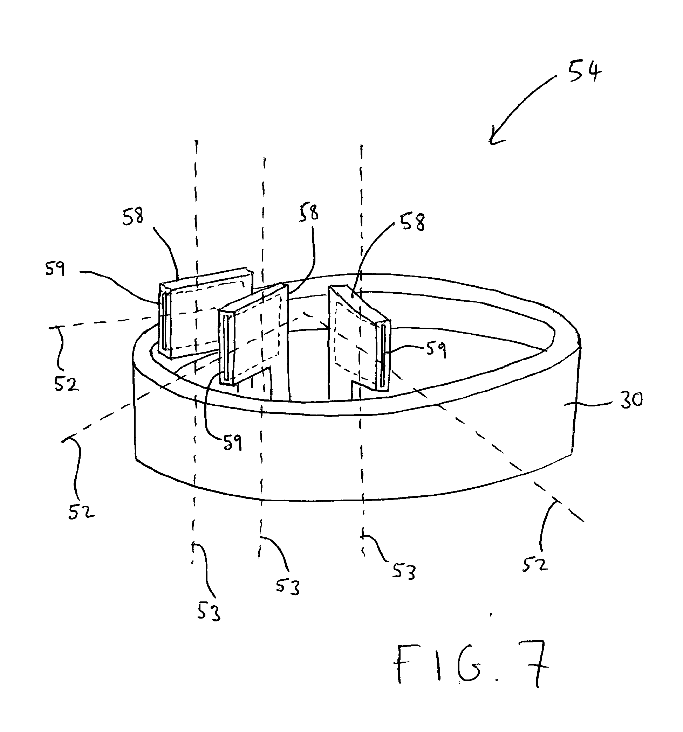

Figure 7 is a schematic illustration (not to scale) of the digital model of the second base plate 54.

In this embodiment, the digital model of the second base plate 54 comprises a digital representation of a plate which is the same shape as the first base plate (which, for convenience and ease of understanding is indicated in Figure 7 by the reference numeral corresponding to the first base plate, i.e. reference numeral 38) and three projections 58.

The longitudinal axes 52 and transverse axes 53 are also shown in Figure 7.

In this embodiment, each of the projections 58 comprises a substantially cuboid-shaped first portion, and a second portion that connects the first portion of that projection 58 to a top surface of the first base plate-shaped portion of the second base plate 54. The first portion of each of the projections 58 is substantially hollow and comprises a slit opening 59 at one end. Each of the projections 58 is connected to and extends away from the top surface of the first base plate-shaped portion of the second base plate 54.

In this embodiment, the interior of the first portion of each of the projections 58 is substantially the same size and shape as a respective region 48.

In this embodiment each of the projections 58 is positioned such that a longitudinal axis of the first portion of that projection 58 is aligned with (i.e. coincident with) a respective longitudinal axis 52. In other words, the longitudinal axis of each of the first portions of the projections 58 points along a different longitudinal axis 52.

In this embodiment each of the projections 58 is positioned such that a transverse axis of the first portion of that projection 58 is aligned with (i.e. coincident with) a respective transverse axis 53. In other words, the transverse axis of each of the first portions of the projections 58 points along a different transverse axis 53.

Furthermore, in this embodiment, each of the projections 58 is such that, were the digital representation of the first plaster cast 40 to be positioned on the top surface of the second base plate 54 (as in the surgery planning model 44), each of the projections 58 would extend substantially up to, but not extend beyond, the top surface of the digital representation of the first plaster cast 40.

In this embodiment, the size and shape of the second base plate 54 is substantially the same as that of the first base plate 38 with the three projections 58 attached thereto.

At step s46, the third computer terminal 22 is used to control the CNC drilling machine 24, such that the CNC drilling machine 24 produces the second base plate 54.

In this embodiment, the second base plate 54 is produced by the CNC drilling machine 24 milling, or drilling, an appropriately shaped piece of plastic or resin into the shape of the second base plate 54, as specified by the digital model of the second base plate 54.

At step s48, the second base plate 54, produced at step s34, is sent (e.g. via post or courier) from CNC drilling facility 14 to the dental laboratory 13.

At step s50, at the dental laboratory, a collar, the first base plate 38, and the first plaster cast 40 are coupled together to form a third assembly.

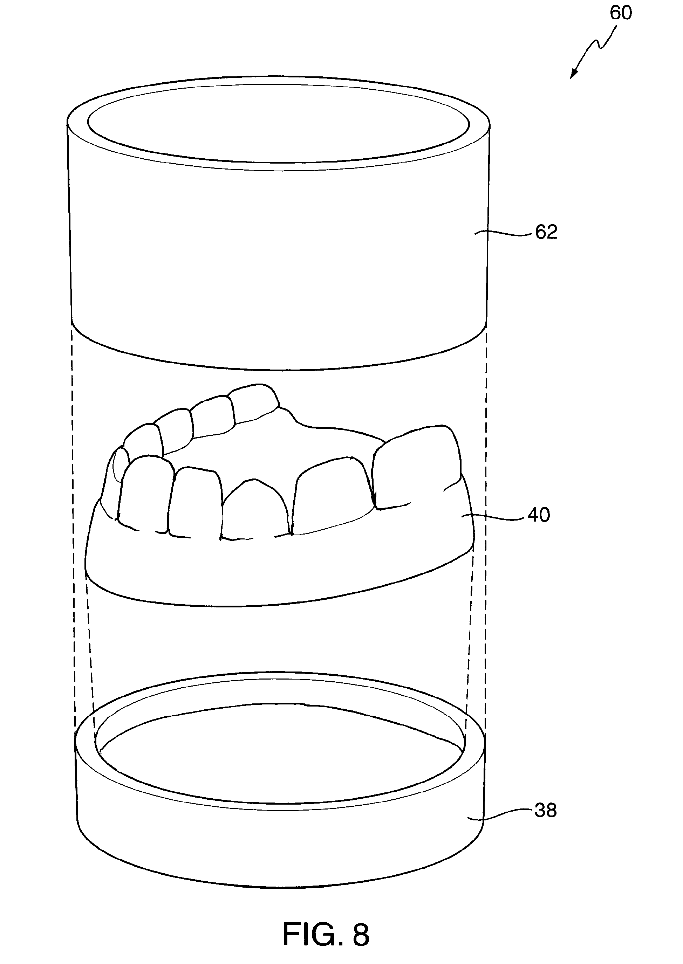

Figure 8 is a schematic illustration (not to scale) of an exploded view of the third assembly 60 formed at step s50 of the process of Figure 3.

In this embodiment, the third assembly 60 comprises the collar 62, the first base plate 38 and the first plaster cast 40.

In this embodiment, the collar 62 is substantially tube-shaped. The external diameter of the collar 62 is substantially equal to the external diameter of the tubular portion of the first base plate 38. Furthermore, the internal diameter of the collar 62 is substantially equal to the internal diameter of the tubular portion of the first base plate 38.

In this embodiment, the first base plate 38 and the first plaster cast 40 are coupled together as described above for the first assembly 36 (and second assembly) with reference to step s18 and Figure 5, i.e. the first plaster cast 40 is positioned on the top surface of the first base plate 38 such that the first plaster cast does not slide across the top surface of the first base plate 38.

In this embodiment, the collar 62 is coupled to the first base plate 38 such that the side of the collar 62 is contiguous with the side of the tubular portion of the first base plate 38. Thus, the collar is placed over the first plaster cast 40.

In this embodiment, the length of the collar 62 is such that, when the collar 62, the first base plate 38 and the first plaster cast 40 are coupled together as described above to form the third assembly 60, the top surface of the first plaster cast 40 does not extend (in a direction away from the first base plate 38) further than the free end of the collar 62.

The above described relative placement of the first base plate 38, the first plaster cast 40, and the fork 26 is indicated in Figure 5 by dotted lines.

At step s52, a silicone rubber cast of a negative impression of the first plaster cast 40, hereinafter referred to as the "silicone rubber negative", is created.

In this embodiment, this is performed by filling the third assembly 60 with liquid silicone rubber and allowing the silicone rubber to solidify. The solidified silicone rubber is then removed from the third assembly 60.

In particular, in this embodiment, liquid silicone rubber is poured into the free end of the collar 62 of the third assembly 60 (i.e. the end of the collar 62 not coupled to the first base plate 38), and onto the first plaster cast 40. This is done until the first plaster cast 40 is completely submersed in liquid silicone rubber. The silicone rubber is then allowed to solidify and is removed from the third assembly 60.

At step s54, the collar 62, the silicone rubber negative (produced at step s52), and second base plate 54 (produced at step s34 and sent to the dental laboratory 13 at step s48) are coupled together to form a fourth assembly.

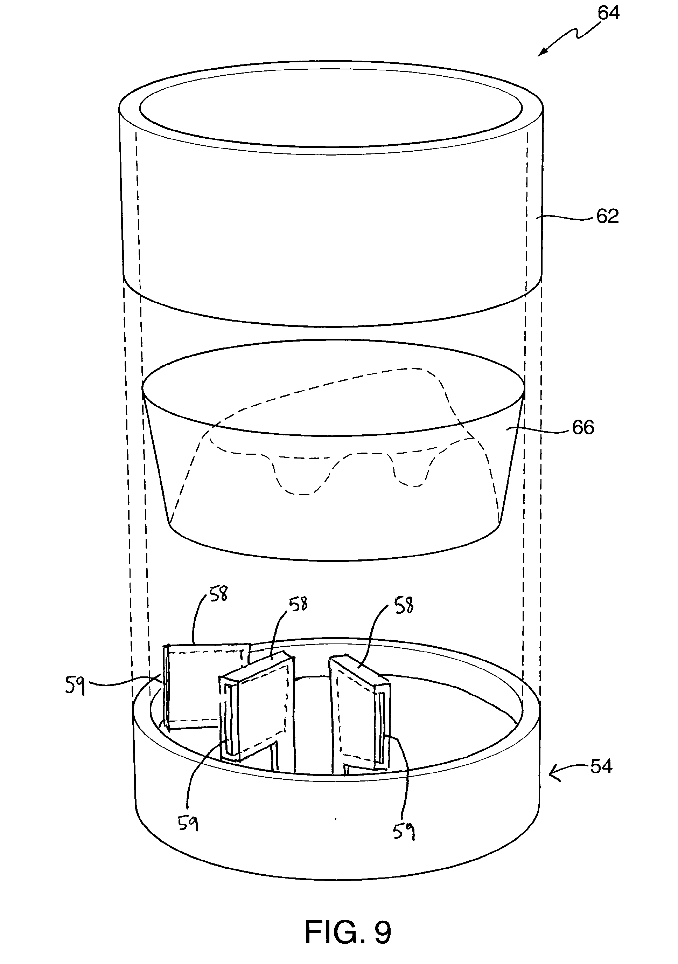

Figure 9 is a schematic illustration (not to scale) of an exploded view of the fourth assembly 64 formed at step s54 of the process of Figure 3.

In this embodiment, the fourth assembly 64 comprises the collar 62, the silicone rubber negative 66 and the second base plate 54. In this embodiment, the collar 62 the silicone rubber negative 66 are coupled together such that the relative position between the collar 62 and the silicone rubber negative 66 in the fourth assembly 64 is substantially the same as the relative position between the collar 62 and the silicone rubber negative 66 when the silicone rubber negative 66 is formed at step s40.

In this embodiment, the collar 62 is coupled to the second base plate 54 such that the side of the collar 62 is contiguous with the side of the tubular portion of the second base plate 54.

Furthermore, in this embodiment the collar 62 is coupled to the second base plate 54 such that, in the fourth assembly 64, the orientation relative to the collar 62 of the portion of the second base plate 54 that is the same shape as the first base plate 38 is substantially the same as the orientation relative to the collar 62 of the first base plate 38 in the third assembly 60.

Equivalently, the fourth assembly 64 may be formed as follows. After the silicon rubber has hardened at step s52, the first base plate 38 and the first plaster cast 40 are removed from the third assembly 60. The relative position between the collar 62 and the silicone rubber negative 66 is maintained. The second base plate 54 is then coupled to the collar 62 such that the portion of the second base plate 54 which is the same shape as the first base plate 38 is in substantially the same position, relative to the collar 62, as the first base plate 38 was before being removed from the third assembly 60.

In this embodiment, the fourth assembly 64 comprises a cavity between the second base plate 54 and the silicone rubber negative 66. This cavity is hereinafter referred to as "the cavity".

At step s56, a second plaster cast is formed by filling the cavity in the fourth assembly 64 with liquid plaster, and allowing this liquid plaster to solidify.

In this embodiment, the cavity may be filled with plaster through a hole in the second base plate 54 (this hole is not shown in the Figures).

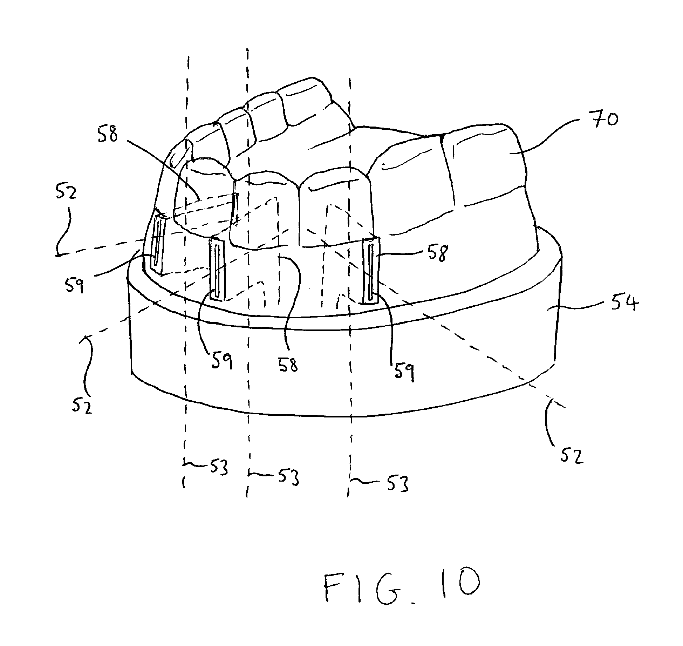

Figure 10 is a schematic illustration (not to scale) showing the second plaster cast 70 formed at step s56. The second plaster cast 70 is coupled to the second base plate 54. The openings 59 of the projections 58 are located at the outer (front) surface of the second plaster cast 70.

In this embodiment, the shape of the second plaster cast 70 is substantially the same as that of the first plaster cast 40 (with the openings 59 to the hollow portions of the projections 58 therein).

In this embodiment, the projections 58 of the second base plate 54 define voids in the second plaster cast 70. In particular, the hollow portions of the projections 58 (which may be accessed via the openings 59) define voids in the second plaster cast 70 (when the second base plate 54 is used to cast the second plaster cast 70). In this embodiment, the second base plate 54 is a mould part for casting the second plaster cast 70 (which, in this embodiment, is a positive cast of the patient's lower jaw bone). In this embodiment, the size, location and the orientation, in the second plaster cast 70, of each of the voids defined by the projections 58 is substantially the same as the size, location and the orientation of a respective region 48 in the surgery planning model 44.

Thus, the second plaster cast 70 is a positive cast of the patient's lower dental arch (as is the first plaster cast 40) and comprises three voids (defined by the projections 58).

At step s58, for each of the projections 58, a metal sheet is placed into the void defined by that projection 58, via the opening 59 of that projection 58.

In this embodiment, the metal sheets are rigid sheets. In some embodiments, one or more of the metal sheets may be made of a different material e.g. a non-metal e.g. a plastic.

In this embodiment, each metal sheet has a thickness and width that is substantially equal to the thickness and width of the void into which that metal sheet has been inserted.

Furthermore, each metal sheet has a length that is larger than the length of the void into which that metal sheet has been inserted, such that each metal sheet protrudes from the void into which that metal sheet has been inserted. In other words, each metal sheet is inserted into a respective projection 58 (via the opening 59 in that projection 58) such that that metal sheet extends away from the top surface of the second plaster cast 70.

Furthermore, in this embodiment, each metal sheet is inserted into a respective projection 58 such that the longitudinal axis of that metal sheet is substantially coincident with the longitudinal axis of that projection 58. Also, each metal sheet is inserted into a respective projection 58 such that the transverse axis of that metal sheet is substantially coincident with the transverse axis of that projection 58.

Thus, after step s58, each of the projections 58 has placed within it a respective metal sheet. Each metal sheet has a non-free end that is positioned within a projection 58, and a free end, opposite to the non-free end, that is distal from the second plaster cast 70

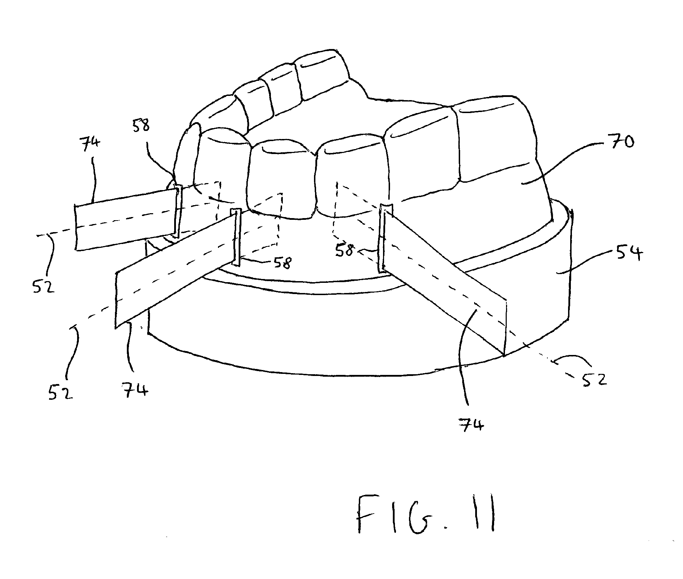

Figure 11 is a schematic illustration (not to scale) showing the second plaster cast 70 coupled to the second base plate 54, and the metal sheets 74 (that have been inserted into the voids defined by the projections 58 via the openings 59).

At step s62, relatively soft resin is applied to the surface of the second plaster cast 70 that corresponds to the lower dental arch of the patient.

For example, at the dental laboratory 13, soft resin is pushed onto the second plaster cast 70 such that the surface of the second plaster cast 70 that corresponds to the lower dental arch of the patient is completely covered by the soft resin.

In this embodiment, the soft resin is applied such that the resin surrounds each of the metal sheets 74 and such that the free ends of the metal sheets are not covered by resin.

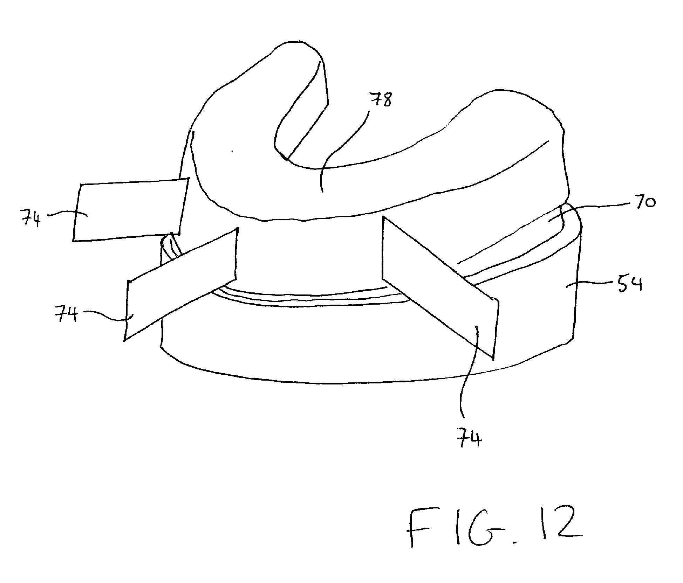

Figure 12 is a schematic illustration (not to scale) of the structure formed at step s62 (i.e. the second plaster cast 70, the second base plate 54, the metal sheets 74, and the 78).

At step s64, the resin 78 applied at step s62 is allowed to harden.

At step s66, the metal sheets 74 are removed from the projections 58.

In this embodiment, each of the metal sheets 74 is removed by pulling that metal sheets 74, from its free end, in a direction that points substantially along the longitudinal axis of that metal sheets 74 and away from the second plaster cast 70, until it is completely removed from the second plaster cast 70 and the second base plate 54.

At step s68, the resin 78 is removed from the second plaster cast 70, thereby providing the guide 2 shown in Figure 1. In this embodiment, the solidified resin 78 forms the guide body 4. The slits 6 in the guide body 4 are those left after removal of the metal sheets 74 from the resin 78.

Thus, the guide 2 is formed.

At step s70, the guide 2 is sent from the dental laboratory 13 to the dental surgery 12 (for use by the dentist).

Thus, a guide, and a method for producing said guide, is provided.

An advantage provided by the above described method for producing a guide is that the slits in the guide are substantially aligned with the regions of the patient's lower jawbone that are to be cut (i.e. during surgery). This tends to facilitate the accurate cutting of the patient's jawbone using e.g. a piezoelectric surgical device.

Also, use of the above described guide tends facilitate the dental surgeon avoiding, for example, nerves or areas of weak bone, when cutting the jawbone of the patient.

An example procedure that may be performed using the above described guide and, for example, a piezoelectric surgical device includes cutting areas of a patient's jawbone, thereby weakening that specific area of the jawbone. Thereafter, orthodontic braces may be fixed to the patient's teeth. The weakening of the specific parts of the patient's jawbone advantageously tends to speed up the movement of the patient's teeth in those areas by the orthodontic braces. Thus, the targeted cutting of the patient's jawbone is provided for.

Further example procedures that may be performed using the above described guide and, for example, a piezoelectric surgical device includes oral surgical procedures such as bone sampling, excision of cysts, third molar extraction, preparation of alveolar sites, creation of an opening into the maxillary sinus (Caldwell Luc), elevation of the maxillary sinus by the crestal route and orthopedic and neurosurgical procedures such as osteoplasty, ostectomy and osteotomy. The above described guide tends to provide for more accurate cutting of the patient's jawbone during such procedures.

The above described method for producing a guide advantageously tends to avoid the use of an X-ray opaque coating on radiological guide, or other structure, placed in the patient's mouth during X-ray. Also, the combination of the use of X-ray and laser scanner images advantageously tends to provide that the images of the patient's teeth in the registered model are of relatively high quality. Thus, it tends to be possible to avoid expensive image filtering processes.

The above described method for producing a guide advantageously tends to avoid the use of stereo lithography (otherwise known as rapid prototyping).

The use of a single fiduciary marker positioned centrally on the fork tends to facilitate the process of registering the images from the different image sources (e.g. as performed at step s38). Different types of structures may be used instead of the fiduciary marker to perform registration of the images from the different image sources, for example three or more tungsten plugs positioned at different points on the fork may be used. However, having such structures at different positions on the fork may mean that capturing images that contain all of the reference structures, and all the patient's teeth, is difficult. Having a single fiduciary marker positioned relatively centrally on the fork tends to alleviate this problem.

The moulding of the guide into the shape of the patient's dental arch (by forming the resin 78, which forms the guide body 4, around the second plaster cast 70) tends to provide that movement of the guide in the patient's mouth during surgery is opposed.

Optionally, the above described guide 2 may be such that the guide comprises one or more additional holes thorough the guide body 4. During use of the guide 2, the guide 2 may be secured to the patient's jawbone by inserting a securing pin, or screw, through each of these holes and into the patient's jawbone. This tends oppose the movement of the guide inside the patient's mouth during surgery. This may, for example, be performed as described in UK Patent Application GB1117955.3, which is incorporated herein by reference. This advantageously tends to provide for the relatively secure fixing of the guide into the mouth of a patient, even if the patient has few (or no) remaining teeth.

What will now be described is a further embodiment of a method of producing a guide 2.

In this embodiment, the CNC drilling facility 14, in addition to comprising the third computer terminal 22 and the CNC drilling machine 24, further comprises a laser scanner (which is hereinafter referred to as the "further laser scanner"). The further laser scanner is substantially the same as the laser scanner 16.

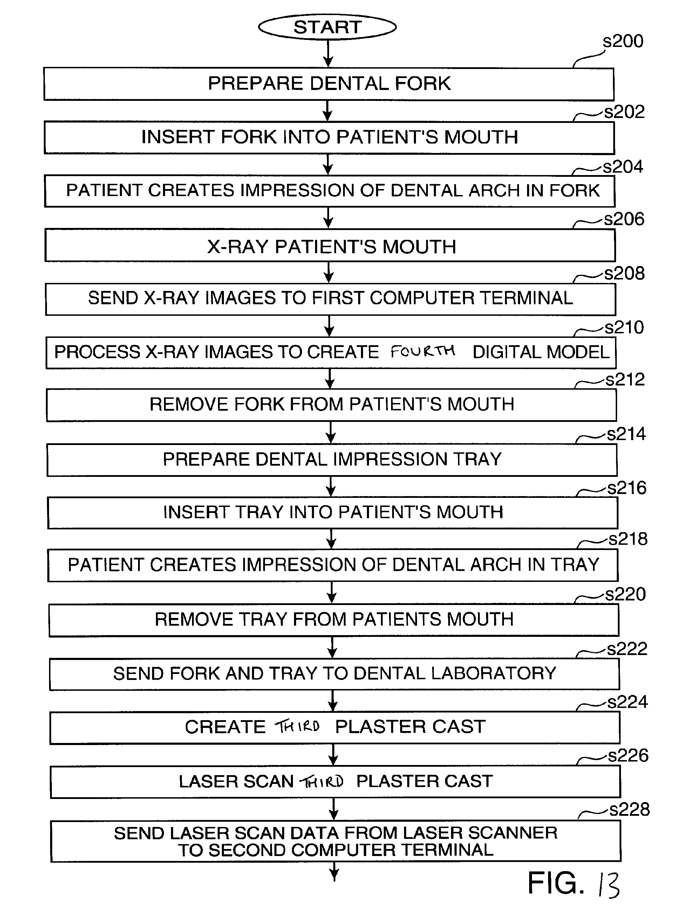

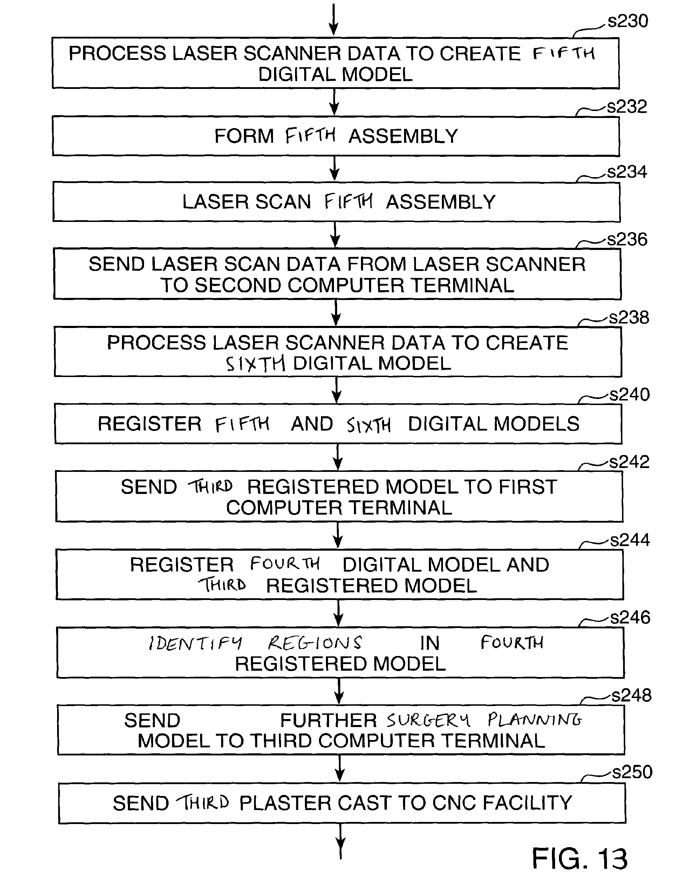

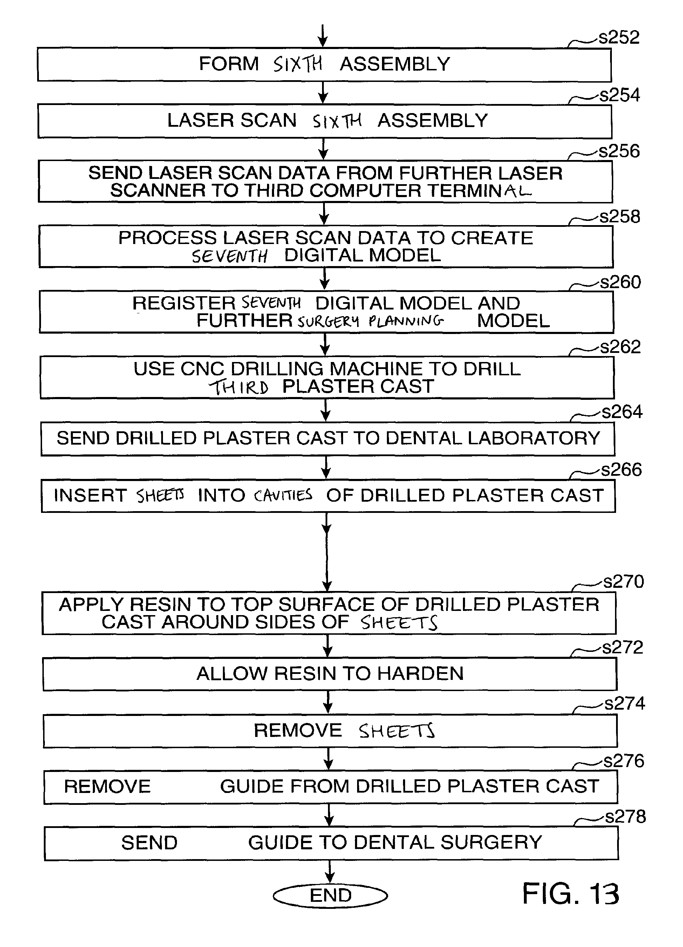

Figure 13 is a process flow chart showing certain steps of a further embodiment of a process of producing the guide.

At step s200, the fork 26 is prepared (in the same way as at step s2 as described above with reference to Figure 3).

At step s202, the prepared fork 26 is inserted into the patient's mouth (in the same way as at step s4 as described above with reference to Figure 3).

At step s204, the patient bites down on the prepared fork 26, thereby creating an impression of the dental arch of the patient in the silicone rubber (in the same way as at step s6 as described above with reference to Figure 3).

At step s206, whilst the fork 26 is still in the patient's mouth and the patient is still biting down on the fork 26, one or more X-ray images of the patient's mouth are taken using the X-ray machine 18 (in the same way as at step s8 as described above with reference to Figure 3).

At step s208, the captured X-ray images (or data corresponding to the X-ray images) are sent from the X-ray machine 18 to the first computer terminal 20 (in the same way as at step s10 as described above with reference to Figure 3).

At step s210, the first computer terminal 20 processes the received X-ray images to create a 3-dimensional digital model of the patient's teeth, the patient's jawbones, and the fork 26 (in the same way as the first digital model is created at step s12 as described above with reference to Figure 3). This 3-dimensional digital model will hereinafter be referred to as the fourth digital model.

At step s212, the fork 26 with attached the silicone rubber is removed from the patient's mouth (in the same way as at step s14 as described above with reference to Figure 3).

At step s214, a dental impression tray (hereinafter referred to as "the tray") is prepared. In this embodiment, the tray is a conventional dental impression tray that may, for example, be customised for the patient. The tray is prepared by applying to a viscous liquid material (e.g. liquid silicone rubber), which sets to become a solid after a certain amount of time.

At step s216, the prepared tray is inserted into the patient's mouth in a conventional manner.

At step s218, the patient bites down on the prepared tray, thereby creating an impression of the dental arch (in particular, the lower dental arch) of the patient in the viscous liquid material that has been applied to the tray.

At step s220, the tray is removed from the patient's mouth. The tray has in it a detailed impression of the (lower) dental arch of the patient.

At step s222, the fork 26 and the tray are sent from the dental surgery 12 to the dental laboratory 13.

At step s224, from the impression of the patient's lower dental arch in the tray, a plaster cast of the patient's lower dental arch, hereinafter referred to as the "third plaster cast", is created at the dental laboratory 13.

This is performed in a similar way to that described above at step s16 for the creation of the first plaster cast from the impression of the lower dental arch in the silicone rubber attached to the bottom surface of the U-shaped portion of the fork 26.

In this embodiment, the third plaster cast is created from the impression of the patient's lower dental arch in the tray. However, in other embodiments, the third plaster cast can be created from the impression of the patient's lower dental arch in the fork 26. In such other embodiments, the use of the tray may be omitted.

At step s226, the third plaster cast is scanned using the laser scanner 16.

In this embodiment, data produced by the scanning of the third plaster cast by the laser scanner 16 are sufficient to allow for a 3-dimensional digital model of the third plaster cast to be created.

At step s228, data from the laser scanner 16 corresponding to the scanned third plaster cast are transferred from the laser scanner 16 to the second computer terminal 17.

At step s230, the second computer terminal 17 processes the received laser scanner data to create a 3-dimensional digital model of the third plaster cast. This 3-dimensional digital model of the third plaster cast is hereinafter referred to as the fifth digital model.

At step s232, the third plaster cast and the fork 26 (with the indented silicone rubber) are coupled together to form a fifth assembly.

In this embodiment, the fifth assembly comprises the third plaster cast and the fork 26 coupled together such that the fork 26 is positioned on to the top of the third plaster cast such that the silicone rubber 42 attached to the bottom surface of the fork 26, into which an indentation of the patient's lower dental arch has been formed, is in contact with the top surface of the third plaster cast (i.e. the surface of the third plaster cast that is the shape of the patient's lower dental arch). Furthermore, this is done such that the (negative) indentations in the silicone rubber 42 mate with the (positive) corresponding protrusions of the patient's lower dental arch on the third plaster cast.

At step s234, the fifth assembly is scanned using the laser scanner 16.

In this embodiment, data produced by the scanning of the fifth assembly by the laser scanner 16 are sufficient to allow for a 3-dimensional digital model of the fifth assembly to be created.

At step s236, data from the laser scanner 16 corresponding to the scanned fifth assembly are transferred from the laser scanner 16 to the second computer terminal 17.

At step s238, the second computer terminal 17 processes the received laser scanner data to create a 3-dimensional digital model of the fifth assembly. This 3-dimensional digital model of the fifth assembly (i.e. the third plaster cast and the fork 26 coupled together as described above) is hereinafter referred to as the sixth digital model.

At step s240, the fifth digital model (created at step s230) and the sixth digital model (generated at step s238) are registered by the second computer terminal 17. In this embodiment, this registration process comprises aligning the digital models using surfaces common to both of those digital models (i.e. in the same way as the second and third digital models were registered at step s34 of Figure 3).

The registered fifth and sixth digital models are hereinafter referred to as the "third registered model", which comprises laser scanned images of the third plaster cast and the fork 26 (with the fiduciary marker 32).

At step s242, the third registered model is sent from the second computer terminal 17 (at the dental laboratory 13) to the first computer terminal 20 (at the dental surgery 12), e.g. via email or over the Internet.

At step s244, the fourth digital model (generated at step s210) and the third registered model (generated at step s240) are registered using the first computer terminal 20. In this embodiment, this registration process is the same as that performed at step s38 of Figure 3 at which step the first digital model and the first registered model are registered, i.e. the image registration performed at step s244 comprises transforming one or more of the models such that the position and orientation of the fiduciary marker 32 in each of the digital models is substantially the same.

The digital model comprising the registered fourth digital model and third registered model is hereinafter referred to as the "fourth registered model".

The image of the fork 26 within the fourth registered model may be removed from the fourth registered model.

At step s246, using the first computer terminal 20, the dental surgeon (or a different party) identifies regions 48 in the fourth registered model that correspond to those regions in the patient's lower jawbone that are to be cut into using the piezoelectric surgical device. This is performed in the same way as performed at step s40 of the process of Figure 3.

The digital model comprising the digital implants 50 positioned in the fourth registered model is hereinafter referred to as the "further surgery planning model".

At step s248, the further surgery planning model is sent from the first computer terminal 20 (at the dental surgery 12) to the third computer terminal 22 (at the CNC drilling facility 14).

At step s250, the third plaster cast is sent from the dental laboratory 13 to the CNC drilling facility 14 (e.g. via courier or post)

At step s252, the third plaster cast and a CNC machine workpiece-holding plate, hereinafter referred to as the "holding plate", are coupled together to form an assembly. This assembly is hereinafter referred to as the sixth assembly. The sixth assembly is placed by securing the third plaster cast onto the holding plate.

In this embodiment, the holding plate is a conventional workpiece-holding plate suitable for use with the CNC drilling machine 24, for example, a pallet of an EROWA(TM) workholding system.

At step s254, the sixth assembly is laser scanned using the further laser scanner (i.e. the laser scanner situated at the CNC drilling facility 14).

In this embodiment, data produced by the scanning of the sixth assembly by the further laser scanner are sufficient to allow for a 3-dimensional digital model of the sixth assembly to be created.

In this embodiment, the sixth assembly is formed and laser scanned (by the further laser scanner) at the CNC drilling facility 14. However, in other embodiments, the sixth assembly may be formed and/or scanned at a different location by different apparatus. For example, the sixth assembly may be formed and scanned (using the laser scanner 16) at the dental laboratory 13.

At step s256, data from the further laser scanner corresponding to the scanned sixth assembly are transferred from the further laser scanner to the third computer terminal 22.

At step s258, the third computer terminal 22 processes the received laser scanner data to create a 3-dimensional digital model of the sixth assembly. This 3-dimensional digital model of the sixth assembly (i.e. the third plaster cast secured to the holding plate) is hereinafter referred to as the seventh digital model.

At step s260, the seventh digital model (created at step s258) and the further surgery planning model (generated at step s246 and sent to the third computer terminal 22 at step s248) are registered by the third computer terminal 22. In this embodiment, this registration process comprises aligning the models using surfaces common to both of those digital models (e.g. surfaces of the digital images of the third plaster cast).

The registered seventh digital model and further surgery planning model is hereinafter referred to as the "fifth registered model". In this embodiment, the fifth registered model comprises digital representations of the patient's jawbone, the third plaster cast, the regions 48, the holding plate, and (optionally) the fork 26. The images of the patient's jawbone may optionally be removed from the fifth registered model.

At step s262, using the fifth registered model, the third computer terminal 22 is used to control the CNC drilling machine 24, such that, the CNC drilling machine 24 drills a plurality of cavities or voids into the third plaster cast. The position, size and orientation of each of the cavities or voids in the third plaster cast is substantially the same as the position, size and orientation of a respective region 48 in the digital third plaster cast (in the fifth registered model).

The third plaster cast with the cavities or voids cut into it is hereinafter referred to as "the drilled plaster cast".

At step s264, the drilled plaster cast, produced at step s262, is sent (e.g. via post or courier) from CNC drilling facility 14 to the dental laboratory 13.

At step s266, a metal sheet 74 is placed into each of the cavities or voids in the drilled plaster cast.

In this embodiment, each metal sheet has a thickness and width that is substantially equal to the thickness and width of the cavity or void in the drilled plaster cast into which that metal sheet 74 has been inserted.

Furthermore, each metal sheet 74 has a length that is larger than the length of the cavity or void into which that metal sheet 74 has been inserted, such that each metal sheet 74 protrudes from the void into which that metal sheet 74 has been inserted. In other words, each metal sheet 74 extends away from the top surface of the drilled plaster cast.

Furthermore, in this embodiment, each metal sheet 74 is inserted into a respective cavity or void in the drilled plaster cast such that the longitudinal axis of that metal sheet 74 is substantially coincident with the longitudinal axis of that cavity or void. Also, each metal sheet 74 is inserted into a respective cavity or void in the drilled plaster cast such that the transverse axis of that metal sheet 74 is substantially coincident with the transverse axis of that cavity or void.

Thus, after step s266, each of the cavities or voids in the drilled plaster cast has placed within it a respective metal sheet 74. Each metal sheet 74 has a non-free end that is positioned within a cavity or void in the drilled plaster cast, and a free end, opposite to the non-free end, that is distal from the drilled plaster cast.

At step s270, relatively soft resin is applied to the surface of the drilled plaster cast.

In this embodiment, the soft resin is applied in a corresponding fashion to that described above with reference to step s62 for the process of Figure 3.

At step s272, the resin applied at step s270 is allowed to harden.

At step s274, the metal sheets 74 are removed from the cavity or void in the drilled plaster cast, and from the hardened resin 78.

At step s276, the hardened resin 78 is removed from the drilled plaster cast. Thus, the guide 2 is formed.

At step s278, the guide 2 is sent from the dental laboratory 13 to the dental surgery 12 (for use by the dentist).

Thus, a further embodiment a method for producing the guide 2 is provided. In addition to many of the advantages mentioned above for the method of Figure 3, a further advantage provided by the above described further embodiment is that the method tends to have fewer steps than conventional methods and so be simpler to perform.

Apparatus, including the computer terminals for implementing the above arrangement, and performing the method steps described above, may be provided by configuring or adapting any suitable apparatus, for example one or more computers or other processing apparatus or processors, and/or providing additional modules. The apparatus may comprise a computer, a network of computers (e.g. a network of computers spread across a number of different locations), or one or more processors, for implementing instructions and using data, including instructions and data in the form of a computer program or plurality of computer programs stored in or on a machine readable storage medium such as computer memory, a computer disk, ROM, PROM etc., or any combination of these or other storage media.

It should be noted that certain of the process steps depicted in the flowcharts of Figures 3 and 13 and described above may be omitted or such process steps may be performed in differing order to that presented above and shown in the Figures. Furthermore, although all the process steps have, for convenience and ease of understanding, been depicted as discrete temporally-sequential steps, nevertheless some of the process steps may in fact be performed simultaneously or at least overlapping to some extent temporally. Furthermore, it should be noted that any of the method steps performed in an above described embodiment may be replaced by any of the method steps from a different embodiment, or those steps from the different embodiment may be performed in addition to the steps of the embodiment in question, such that the same functionality (e.g. the production of the guide) is provided.

In the above embodiments, the patient is an adult male human. However, in other embodiments the patient is a different type of patient. For example, in other embodiments, the patient is a human child and/or a female human. In such embodiments, any of the apparatus may be modified or changed so as to accommodate the different type of patient. For example, a smaller sized fork may be used for a child.

In the above embodiments, the guide has the features described above with reference to Figure 1. Furthermore, in the above embodiments, the dental guide is made of those materials specified above. However, in other embodiments, the dental guide comprises additional features instead of or in addition to those described above (for example, a different number of slits). Furthermore, in other embodiments, the components of the guide are made of different appropriate materials. In other embodiments, the functionality provided by the components of the guide is supplied by one or more different appropriate features.

In the above embodiments, the shapes of the various pieces of apparatus used in the methods (e.g. the shape of the fork or a base plate), and the shapes of the various entities created or produced during the methods (e.g. the shape of a plaster cast) are as specified above. However, in other embodiments the shape of one or more of the various pieces of apparatus and/or one or more of the various entities is different to that described above such that the above described functionality is provided.

In the above embodiments, the materials of which the various pieces of apparatus used in the methods are made (e.g. resin of the fork or a base plate), and the materials used to create or produce the various entities created or produced during the methods (e.g. the plaster used to create a plaster cast, or the silicone rubber used to create a silicone rubber negative) are as specified above. However, in other embodiments the materials of which one or more of the various pieces of apparatus are made and/or the materials used to make one or more of the various entities is different to that described above such that the above described functionality is provided.

In the above embodiments, certain of the method steps are performed at a specified location (e.g. either the dental surgery of the CNC drilling facility) and/or by a specified party (e.g. by the dental surgeon or an assistant to the dental surgeon). However, in other embodiments a method step may be performed at a different location or locations. Also, in other embodiments a method step may be performed by a different party or parties.

In the above embodiments, images are generated using an X-ray machine, or using a laser scanner. Images from these two image sources are then registered to create a registered model. However, in other embodiments, instead of or in addition to one or both of the X-ray machine and the laser scanner, one or more different types of imaging device is used to create appropriate images. The images from any of the image sources may then be registered (e.g. if they are of good quality) to create a registered model. For example, in other embodiments, a CT scanning machine, an MRI machine and/or an ultrasound machine is used instead of or in addition to the X-ray machine to capture images of the internal structure of the portion of the dental arch of the patient. For example, in other embodiments, a contact 3D scanner and/or CMM machine is used instead of or in addition to the laser scanner to create the digital model of the physical model of the dental arch.

In the above embodiments, there are three regions in the second registered model that correspond to those regions in the patient's lower jawbone that are to be cut into using the piezoelectric surgical device. However, in other embodiments, there are a different number of such regions. Thus, in some embodiments, there are a different number of projections from the second base plate to that described above. Thus, in some embodiments, there are a different number of slits in the guide to that described above.

In the above embodiments, the regions in the second registered model (that correspond to those regions in the patient's lower jawbone that are to be cut into using the piezoelectric surgical device) are as described above. However, in other embodiments, one or more of the regions may have a different size, shape, position and/or orientation in the second registered model to that described above. Thus, in some embodiments, one or more of the projections from the second base plate may have a different size, shape, position and/or orientation in the second base plate to that described above. Thus, in some embodiments, one or more of the slits in the guide may have a different size, shape, position and/or orientation in the guide to that described above.

In some embodiments, regions that are to be cut using a piezoelectric surgical device are identified in a digital model prior to registering that model with a different model. In other embodiments, images from only one image source are used to construct the digital model in which regions that are to be cut using a piezoelectric surgical device are identified.

In the above embodiments, images are registered together using the fiduciary marker on the fork as a reference. In the above embodiments, the fiduciary marker is a 3D bar code. In the above embodiments, the fiduciary marker is positioned relatively centrally on the fork. However, in other embodiments the images from the multiple image sources may be registered in a different way, for example, using a different reference point or set of reference points. In other embodiments, the fiduciary marker is a different type of feature or set of features, for example, a set of three or more tungsten (or other radio-opaque material) plugs may be positioned at different respective points in the body of the fork. These plugs may then be used to register images from the different image sources. In other embodiments, the fiduciary marker is positioned at a different point on the fork.

In the above embodiments, a base plate comprising one or more projections is produced using a CNC drilling process based on a digital model. However, in other embodiments such base plates are produced by a different appropriate method, for example, stereo lithography or rapid prototyping.

In this embodiment, the guide is for facilitating dental surgery including the cutting of a lower jawbone of a patient. However, the guide is for facilitating a different type of surgery, e.g. non-dental surgery, e.g. non dental piezoelectric surgery. For example, in some embodiments, an above described method may be performed with the jaw or dental arch of a patient being replaced by a different part of the patient's body, for example a hand or foot of the patient. Thus, a guide for placing over a different part of the patient's body (e.g. a hand or foot) may be produced. This guide may be used to guide the cutting head of a piezoelectric surgical device during a surgical procedure performed on that part of the patient's body.

Further aspects of the invention are provided by the subject matter of the following clauses:
1. A surgical guide comprising:
   a guide body configured to fit to a surface of a part of a human or animal body; and
   a cutting head guide that, when the surgical guide is fitted to the surface of the part of a human or animal body, defines a predetermined path across the surface from one point on the surface to a different point on the surface, the cutting head guide being for guiding a cutting head of a surgical device along the defined path.
2. A surgical guide according to clause 1, wherein the part of a human or animal body is a dental arch of the human or animal.
3. A surgical guide according to clause 1, wherein the part of a human or animal body is a part of a human or animal body other than a dental arch of the human or animal.
4. A surgical guide according to any of clauses 1 to 3, wherein the surgical device is a piezoelectric surgical device.
5. A surgical guide according to any of clauses 1 to 3, wherein the surgical device is a surgical device other than a drill.
6. A surgical guide according to any of clauses 1 to 5, wherein:
   the cutting head guide comprises a channel through the guide body, the channel being such that, when the surgical guide is fitted to the surface of the part of a human or animal body, a cutting head of a surgical device may be moved along the channel from a first position to a second position;
   the first position is a position in which the cutting head of a surgical device is not in contact with the surface of the part of a human or animal body; and
   the second position is a position in which the cutting head of a surgical device is in contact with the surface of the part of a human or animal body.
7. A surgical guide according to clause 6, wherein the channel is such that, when the surgical guide is fitted to the surface of the part of a human or animal body and when the cutting head of a surgical device is in the second position, the cutting head of a surgical device may be moved along the path guided by the channel.
8. A surgical guide according to clause 6 or 7, wherein the channel has non-round cross-section in a plane through which the cutting head is moved when the cutting head is moved along the path.
9. A surgical guide according to any of clauses 6 to 8, wherein the channel is a slit in the surgical guide.
10. A surgical guide according to clause 9, wherein each slit is a substantially straight slit.
11. A surgical guide according to any of clauses 1 to 10, wherein the guide body comprises a structure that is complementary to the surface of a part of a human or animal body.
12. A surgical guide comprising:
   a guide body configured to fit to a part of a human or animal body; and
   a channel through the guide body; wherein
   the channel is such that, when the surgical guide is fitted to the surface of the part of a human or animal body, a cutting head of a surgical device may be moved along the channel from a first position to a second position;
   the first position is a position in which the cutting head of a surgical device is not in contact with the part of a human or animal body;
   the second position is a position in which the cutting head of a surgical device is in contact with the part of a human or animal body; and
   the channel has a non-round cross section in the plane that is perpendicular to the direction in which the cutting head of a surgical device moves when the cutting head of a surgical device is moved along the channel from a first position to a second position.
13. A surgical guide according to clause 12, wherein the part of a human or animal body is a dental arch of the human or animal.
14. A surgical guide according to clause 12, wherein the part of a human or animal body is a part of a human or animal body other than a dental arch of the human or animal.
15. A surgical guide according to any of clauses 12 to 14, wherein the surgical device is a piezoelectric surgical device.
16. A surgical guide according to any of clauses 12 to 15, wherein the surgical device is a surgical device other than a drill.
17. A surgical guide according to any of clauses 12 to 16, wherein, when the surgical guide is fitted to the part of a human or animal body, the channel defines a predetermined path across the surface of the part of a human or animal body from one point on the surface to a different point on the surface, and the channel is for guiding, along the defined path, a cutting head of a surgical device that has been moved along the channel from the first position to the second position.
18. A surgical guide according to any of clauses 12 to 17, wherein the channel is a slit in the guide body.
19. A surgical guide according to clause 18, wherein each slit is a substantially straight slit.
20. A surgical guide according to any of clauses 12 to 19, wherein the guide body comprises a structure that is complementary to the surface of a part of a human or animal body.
21. A method of manufacturing a surgical guide according to any of clauses 1 to 15, the method comprising:
   providing an assembly, the assembly comprising:
      a first positive physical model of a part of a human or animal body, the first positive physical model comprising a location indicating structure corresponding to a predetermined location in the part of a human or animal body; and
      a location indicating member coupled to the location indicating structure;
   coating at least part of the assembly with a coating material;
   removing, from the coated assembly, the location indicating member; and
   removing, from the first positive physical model, the coating material, thereby producing the surgical guide.
22. A method according to clause 21, wherein the location indicating structure corresponds to a predetermined path across a surface of the part of a human or animal body.
23. A method according to clause 21 or 22, wherein the part of a human or animal body is at least part of a dental arch of the human or animal.
24. A method according to clause 21 or 22, wherein the part of a human or animal body is part of the human or animal body other than a dental arch of the human or animal.
25. A method according to any of clauses 21 to 24, wherein the location indicating structure is any location indicating structure other than a bore.
26. A method according to any of clauses 21 to 25, wherein the step of providing the assembly comprises:
   providing the first positive physical model; and
   coupling, to the location indicating structure of the first positive physical model, a location indicating member.
27. A method according to clause 26, wherein
   the location indicating structure is a cavity located in the first positive physical model at a location that corresponds to the predetermined location in the part of a human or animal body; and
   the step of coupling comprises inserting the location indicating member into the cavity.
28. A method according to clause 27, wherein
   the cavity is a slit in the first positive physical model; and
   the location indicating member is a sheet of material.
29. A method according to any of clauses 21 to 28, wherein the step of providing the first positive physical model comprises:
   producing a first digital model, the first digital model being a digital model of the internal structure of the part of a human or animal body;
   specifying, in the digital model, the predetermined location in the part of a human or animal body, thereby producing a second digital model;
   producing a first negative physical model of the part of a human or animal body;
   using the first negative physical model, producing a second positive physical model of the part of a human or animal body;
   producing a third digital model, the third digital model being a digital model of the second positive physical model;
   registering the second digital model and the third digital model, thereby producing a fourth digital model; and
   using the fourth digital model, producing the first positive physical model.
30. A method according to clause 29, wherein the step of, using the fourth digital model, producing the first positive physical model comprises:
   using the fourth digital model, controlling a cutting machine to cut the second positive physical model so as to form the one or more location indicating structures in the second positive physical model, thereby producing the first positive physical model.
31. A method according to clause 29, wherein the step of, using the fourth digital model, producing the first positive physical model comprises:
   using the fourth digital model, producing a mould for the first positive physical model; and
      using the mould, producing the first positive physical model.
32. A method according to clause 31, wherein:
   the mould comprises a first mould part and a second mould part; and
   the step of producing the mould comprises:
      producing a second negative physical model of the part of a human or animal body, thereby providing the first mould part; and
      using the fourth digital model, producing the second mould part; and
      the second mould part comprises:
         a base portion; and
         one or more projections extending from the base portion, each projection defining a respective location indicating structure in the first positive physical model.
33. A method according to clauses 32, wherein each projection is any projection other than a hollow tube.
34. A method according to any of clauses 29 to 33, wherein the step of producing a first digital model comprises:
   capturing one or more X-ray images of the part of a human or animal body; and
   producing the digital model using said one or more X-ray images.
35. A method according to any of clauses 29 to 34, wherein the step of producing the first negative physical model comprises direct impression modelling of the part of a human or animal body.
36. A mould part for a mould, the mould being for producing a positive physical model of part of a human or animal body, the positive physical model comprising one or more location indicating structures, the location of each of the location indicating structure in the positive physical model being substantially the same as a respective predetermined location in the part of the human or animal body, the mould part comprising:
   a base portion; and
   one or more projections extending from the base portion; wherein
   each projection defines a respective location indicating structure in positive physical model.
37. A mould part according to clause 36, wherein each location indicating structure corresponds to a predetermined path across a surface of the part of a human or animal body.
38. A mould part according to clause 36 or 37, wherein each predetermined location in the part of the human or animal body is a predetermined path across a surface of the part of a human or animal body.
39. A mould part according to any of clauses 36 to 38, wherein the part of the human or animal body is at least part of a dental arch of the patient.
40. A mould part according to any of clauses 36 to 38, wherein the part of a human or animal body is part of the human or animal body other than a dental arch of the patient.
41. A mould part according to any of clauses 36 to 40, wherein each predetermined location in the part of the human or animal body is a location in the part of the human or animal body at which the part of the human or animal body is to be cut using a surgical device.
42. A mould part according clause 41, wherein the surgical device is any surgical device other than a dental drill.
43. A mould part according to clause 41 or 42, wherein the surgical device is a piezoelectric surgical device.
44. A mould part according to any of clauses 36 to 43, wherein each location indicating structure is any location indicating structure other than a bore.
45. A mould part according to any of clauses 36 to 44, wherein each projection is any projection other than a hollow tube.
46. A mould part according to any of clauses 36 to 45, wherein one or more of the projections comprise respective cavities, each cavity being accessible via an opening, such that each projection is adapted to receive a location indicating member.
47. A mould part according to clause 46, wherein one or more of the openings is a slit.
48. A mould part according to clause 47, wherein each slit is a substantially straight slit.
49. A method of manufacturing a mould part according to any of clauses 36 to 48, the method comprising:
   producing a first digital model, the first digital model being a digital model of the internal structure of the part of the human or animal body;
   specifying, in the digital model, each of the predetermined locations in the part of the human or animal body, thereby producing a second digital model;
   producing a negative physical model of the part of the human or animal body;
   using the negative physical model, producing a positive physical model of the at part of the human or animal body;
   producing a third digital model, the third digital model being a digital model of the positive physical model;
   registering the second digital model and the third digital model, thereby producing a fourth digital model; and
   using the fourth digital model, controlling a cutting machine to cut a work piece into the shape of the mould part.
50. A method according to clause 49, wherein the part of the human or animal body is at least part of a dental arch of the patient.
51. A method according to clause 49, wherein the part of a human or animal body is part of the human or animal body other than a dental arch of the patient.
52. A method according to any of clauses 49 to 51, wherein the step of producing a first digital model comprises:
   capturing one or more X-ray images of the part of the human or animal body; and
   producing the digital model using said one or more X-ray images.
53. A method according to any of clauses 49 to 52, wherein the step of producing the negative physical model comprises direct impression modelling of the part of the human or animal body.
54. A method of manufacturing a surgical guide, the method comprising:
   providing a mould for producing a positive physical model of part of a human or animal body, the mould comprising:
      a mould part according to any of clauses 30 to 40; and
      a further mould part, the further mould part being a negative physical model of the part of the human or animal body;
   using the mould, producing a positive physical model of the part of the human or animal body, the positive physical model comprising one or more location indicating structures, the location of each of the location indicating structure in the positive physical model being substantially the same as a respective predetermined location in the part of the human or animal body;
   coupling, to each of the location indicating structures of the first positive physical model, a respective location indicating member, thereby providing an assembly;
   coating at least part of the assembly with a coating material;
   removing, from the coated assembly, the one or more indicating members; and
   removing, from the positive physical model, the coating material, thereby producing the surgical guide.
55. A method according to clause 54, wherein the part of the human or animal body is at least part of a dental arch of the patient.
56. A method according to clause 54, wherein the part of the human or animal body is part of the human or animal body other than a dental arch of the patient.
57. A method according to any of clauses 54 to 46, wherein the step of providing a mould part comprises manufacturing the mould part using a method according to any of clauses 49 to 53.
58. A method of manufacturing a surgical guide, the method comprising:
   producing a first digital model, the first digital model being a digital model of the internal structure of part of the human or animal body;
   specifying, in the digital model, one or more predetermined locations in the part of the human or animal body, thereby producing a second digital model;
   producing a positive physical model of the part of the human or animal body;
   producing a third digital model, the third digital model being a digital model of the positive physical model;
   registering the second digital model and the third digital model, thereby producing a fourth digital model;
   using the fourth digital model, controlling a cutting machine to cut the positive physical model so as to form one or more location indicating structures in the positive physical model, each location indicating structure in the positive physical model corresponding to a respective one of the predetermined locations in the part of the human or animal body;
   coupling, to each of the location indicating structures formed in the first positive physical model, a respective location indicating member, thereby providing an assembly;
   coating at least part of the assembly with a coating material;
   removing, from the coated assembly, the one or more indicating members; and
   removing, from the positive physical model, the coating material, thereby producing the surgical guide.
59. A method according to clause 58, wherein specifying, in the digital model, one or more predetermined locations in the part of the human or animal body comprises specifying, in the digital model, one or more paths across a surface of the part of a human or animal body.
60. A method according to clause 58 or 59, wherein the part of the human or animal body is at least part of a dental arch of the patient.
61. A method according to clause 58 or 59, wherein the part of the human or animal body is part of the human or animal body other than a dental arch of the patient.
62. A method according to any of clauses 58 to 61, wherein the step of producing a positive physical model of the part of the human or animal body comprises:
   producing a negative physical model of the at least part of the part of the human or animal body; and
   using the negative physical model, producing the positive physical model.
63. A method according to any of clauses 58 to 62, wherein each predetermined location in the part of the human or animal body is a location in the part of the human or animal body at which the part of the human or animal body is to be cut using a surgical device.
64. A method according to clause 63, wherein the surgical device is any surgical device other than a dental drill.
65. A method according to clause 63 or 64, wherein the surgical device is a piezoelectric surgical device.
66. A method according to any of clauses 58 to 65, wherein each location indicating structure is any location indicating structure other than a bore.
67. A method according to any of clauses 58 to 66, wherein one or more of the location indicating structures comprise respective cavities, each cavity being accessible via an opening, such that each cavity is adapted to receive a location indicating member.
68. A method according to clause 67, wherein the step of coupling, to each of the location indicating structures formed in the first positive physical model, a respective location indicating member comprises inserting a location indicating member into each of the one or more cavities.
69. A method according to clause 67 or 68, wherein one or more of the openings is a slit.
70. A method according to clause 69, wherein each slit is a substantially straight slit.
71. A method according to clause 69 or 70, wherein, for each slit, the location indicating member that is inserted into that slit is a sheet of material.

## Claims

1. A surgical guide comprising:
a guide body configured to fit to a part of a human or animal body; and
a channel through the guide body; wherein
the channel is such that, when the surgical guide is fitted to the surface of the part of a human or animal body, a cutting head of a surgical device may be moved along the channel from a first position to a second position;
the first position is a position in which the cutting head of a surgical device is not in contact with the part of a human or animal body;
the second position is a position in which the cutting head of a surgical device is in contact with the part of a human or animal body; and
the channel has a non-round cross section in the plane that is perpendicular to the direction in which the cutting head of a surgical device moves when the cutting head of a surgical device is moved along the channel from a first position to a second position.

2. A surgical guide according to claim 1, wherein, when the surgical guide is fitted to the part of a human or animal body, the channel defines a predetermined path across the surface of the part of a human or animal body from one point on the surface to a different point on the surface, and the channel is for guiding, along the defined path, a cutting head of a surgical device that has been moved along the channel from the first position to the second position.

3. A surgical guide comprising:
a guide body configured to fit to a surface of a part of a human or animal body; and
a cutting head guide that, when the surgical guide is fitted to the surface of the part of a human or animal body, defines a predetermined path across the surface from one point on the surface to a different point on the surface, the cutting head guide being for guiding a cutting head of a surgical device along the defined path.

4. A surgical guide according to any of claims 1 to 3, wherein
the part of a human or animal body is a dental arch of the human or animal;
the guide body comprises a structure that is complementary to the surface of a part of a human or animal body
the surgical device is a piezoelectric surgical device; and
the channel is a substantially straight slit in the surgical guide.

5. A method of manufacturing a surgical guide according to any of claims 1 to 4, the method comprising:
providing an assembly, the assembly comprising:
a first positive physical model of a part of a human or animal body, the first positive physical model comprising a location indicating structure corresponding to a predetermined location in the part of a human or animal body; and
a location indicating member coupled to the location indicating structure;
coating at least part of the assembly with a coating material;
removing, from the coated assembly, the location indicating member; and
removing, from the first positive physical model, the coating material, thereby producing the surgical guide.

6. A method according to claim 5, wherein
the step of providing the assembly comprises:
providing the first positive physical model; and
coupling, to the location indicating structure of the first positive physical model, a location indicating member;
the location indicating structure is a cavity located in the first positive physical model at a location that corresponds to the predetermined location in the part of a human or animal body;
the step of coupling comprises inserting the location indicating member into the cavity.
the cavity is a slit in the first positive physical model; and
the location indicating member is a sheet of material.

7. A method according to claim 5 or 6, wherein the step of providing the first positive physical model comprises:
producing a first digital model, the first digital model being a digital model of the internal structure of the part of a human or animal body;
specifying, in the digital model, the predetermined location in the part of a human or animal body, thereby producing a second digital model;
producing a first negative physical model of the part of a human or animal body;
using the first negative physical model, producing a second positive physical model of the part of a human or animal body;
producing a third digital model, the third digital model being a digital model of the second positive physical model;
registering the second digital model and the third digital model, thereby producing a fourth digital model; and
using the fourth digital model, producing the first positive physical model.

8. A mould part for a mould, the mould being for producing a positive physical model of part of a human or animal body, the positive physical model comprising one or more location indicating structures, the location of each of the location indicating structure in the positive physical model being substantially the same as a respective predetermined location in the part of the human or animal body, the mould part comprising:
a base portion; and
one or more projections extending from the base portion; wherein
each projection defines a respective location indicating structure in positive physical model.

9. A mould part according to claim 8, wherein
each location indicating structure corresponds to a predetermined path across a surface of a dental arch of a human;
each predetermined location in the part of the human or animal body is a location in the part of the human or animal body at which the part of the human or animal body is to be cut using a piezoelectric surgical device;
one or more of the projections comprise respective cavities, each cavity being accessible via an opening, such that each projection is adapted to receive a location indicating member; and
one or more of the openings is a slit.

10. A method of manufacturing a mould part, the mould part according to claims 8 or 9, the method comprising:
producing a first digital model, the first digital model being a digital model of the internal structure of the part of the human or animal body;
specifying, in the digital model, each of the predetermined locations in the part of the human or animal body, thereby producing a second digital model;
producing a negative physical model of the part of the human or animal body;
using the negative physical model, producing a positive physical model of the at part of the human or animal body;
producing a third digital model, the third digital model being a digital model of the positive physical model;
registering the second digital model and the third digital model, thereby producing a fourth digital model; and
using the fourth digital model, controlling a cutting machine to cut a work piece into the shape of the mould part.

11. A method according to claim 10, wherein
the step of producing a first digital model comprises capturing one or more X-ray images of the part of the human or animal body, and producing the digital model using said one or more X-ray images; and
the step of producing the negative physical model comprises direct impression modelling of the part of the human or animal body.

12. A method of manufacturing a surgical guide, the method comprising:
providing a mould for producing a positive physical model of part of a human or animal body, the mould comprising:
a mould part according to claim 8 or 9; and
a further mould part, the further mould part being a negative physical model of the part of the human or animal body;
using the mould, producing a positive physical model of the part of the human or animal body, the positive physical model comprising one or more location indicating structures, the location of each of the location indicating structure in the positive physical model being substantially the same as a respective predetermined location in the part of the human or animal body;
coupling, to each of the location indicating structures of the first positive physical model, a respective location indicating member, thereby providing an assembly;
coating at least part of the assembly with a coating material;
removing, from the coated assembly, the one or more indicating members; and
removing, from the positive physical model, the coating material, thereby producing the surgical guide.

13. A method according to claim 12, wherein the step of providing a mould part comprises manufacturing the mould part using a method according to claim 10 or 11.

14. A method of manufacturing a surgical guide, the method comprising:
producing a first digital model, the first digital model being a digital model of the internal structure of part of the human or animal body;
specifying, in the digital model, one or more predetermined locations in the part of the human or animal body, thereby producing a second digital model;
producing a positive physical model of the part of the human or animal body;
producing a third digital model, the third digital model being a digital model of the positive physical model;
registering the second digital model and the third digital model, thereby producing a fourth digital model;
using the fourth digital model, controlling a cutting machine to cut the positive physical model so as to form one or more location indicating structures in the positive physical model, each location indicating structure in the positive physical model corresponding to a respective one of the predetermined locations in the part of the human or animal body;
coupling, to each of the location indicating structures formed in the first positive physical model, a respective location indicating member, thereby providing an assembly;
coating at least part of the assembly with a coating material;
removing, from the coated assembly, the one or more indicating members; and
removing, from the positive physical model, the coating material, thereby producing the surgical guide.

15. A method according to claim 14, wherein
specifying, in the digital model, one or more predetermined locations in the part of the human or animal body comprises specifying, in the digital model, one or more paths across a surface of the part of a human or animal body; and
the part of the human or animal body is at least part of a dental arch of the patient.
